# EUROPEAN PATENT APPLICATION

(11) **EP 3 851 519 A1**
(43) Date of publication of application: **21.07.2021**
(21) Application number: 19866268.6
(22) Date of filing: 27.09.2019
(51) Int. Cl.: C12N 5/077, A61K 35/28, A61K 38/17, A61K 38/18, A61K 38/19, A61K 38/20, A61P 9/10, A61P 29/00, C07C 251/86, C12P 21/02

(54) **ADDITIVE FOR MEDIUM FOR PROMOTING PRODUCTION OF PARACRINE FACTOR**

(30) Priority: 28.09.2018 JP 2018185799
(71) Applicant: Nissan Chemical Corporation, Tokyo 103-6119 (JP)
(72) Inventor: ANNO, Shiho, Shiraoka-shi, Saitama 349-0294 (JP); FUKASAWA, Natsuki, Shiraoka-shi, Saitama 349-0294 (JP); NISHINO, Taito, Shiraoka-shi, Saitama 349-0294 (JP)
(74) Representative: J A Kemp LLP
(86) International application number: PCT/JP2019/038267
(87) International publication number: WO 2020/067477

(57) **Abstract**

The present invention provides a medium additive for promoting production of a paracrine factor in a cell, containing a compound represented by the following formula (I) or a salt thereof: 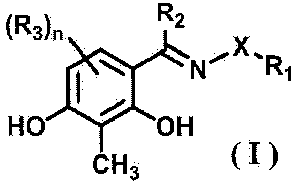 wherein each symbol is as defined in the DESCRIPTION.

## Description

### [Technical Field]

The present invention relates to a medium additive for promoting production of a paracrine factor, a medium added with the medium additive, and a method using the medium for producing a cell showing promoted production of a paracrine factor. In addition, the present invention relates to a therapeutic agent or composition for transplantation for an inflammatory disease or an ischemic disease, the agent or composition containing a cell produced by the production method of the cell. Furthermore, the present invention relates to a method for producing a paracrine factor, including culturing cells in a medium added with the medium additive.

### [Background Art]

In recent years, the development of pharmaceutical products using living cells or tissues and research on regenerative medicine have progressed and are attracting attention. In particular, research on organ regeneration technology using pluripotent stem cells such as ES cell, iPS cell and the like has been accelerated. On the other hand, a cell therapy using somatic stem cells such as mesenchymal stem cells (hereinafter sometimes to be referred to as "MSC"), bone marrow stem cells and the like utilizes the inherent functions of somatic stem cell to repair a tissue damaged by a disease. It is attracting attention as a more feasible therapy in regenerative medicine, and the research is underway.

With regard to the mechanism of MSC transplantation therapy, plural action mechanisms have been assumed. As shown in non-patent document 1 and non-patent document 2, a mechanism is known where the repair function in vivo is promoted by the paracrine effect of the transplanted MSCs. Examples of the paracrine effect of MSC showing a therapeutic effect include angiogenic action, anti-inflammatory action and the like due to the secretion of paracrine factors such as cytokine, growth factor, and the like.

In recent years, it has also become clear that various properties, stimulus responsiveness, cell function, and the like are different between the cells of a single layer culture system and the cells of in vivo tissues. Accordingly, the demand for 3D culture, particularly spheroid culture, which forms a 3D structure close to the in vivo tissue structure, is increasing than single layer culture in which a single layer structure is formed.

Regarding MSC, there is a report that the paracrine effect is enhanced by spheroid culture. Non-patent document 3 describes that spheroid-cultured MSCs showed an increase in the secretion of anti-inflammatory proteins TNF alpha induced protein 6 (hereinafter TSG-6) and Stanniocalcin-1 (hereinafter STC-1). In addition, non-patent document 4 describes that the production amount of VEGF (Vascular endothelial growth factor) and FGF-2 (Fibroblast growth factor-2), which are angiogenesis-promoting proteins, is increased in spheroid-cultured MSC, and further that angiogenesis was promoted and necrosis was improved by transplanting MSC spheroids into the ischemic limbs of mice.

For the transplantation therapy of MSC spheroid, various culture methods are being developed for the production of spheroids with higher therapeutic effect. Patent document 1 describes that spheroidization of MSC by using a biocompatible polymer block increased the secretion amount of anti-inflammatory protein and the like and improved inflammatory diseases.

### [Document List]

### [Patent document]

patent document 1: WO 2017/221879

### [Non-patent documents]

non-patent document 1: Gnecchi M et al, Circ Res. 2008 Nov 21; 103 (11) :1204-19.
non-patent document 2: Madrigal M et al, J Transl Med. 2014 Oct 11; 12:260.
non-patent document 3: Bartosh TJ et al, Proc Natl Acad Sci U S A. 2010 Aug 3; 107(31):13724-9.
non-patent document 4: Bhang SH et al, Tissue Eng Part A. 2012 Oct; 18(19-20):2138-47.

### [Summary of Invention]

### [Technical Problem]

Methods for treating a disease utilizing the paracrine effect of cells, including the aforementioned example of MSC, can be very effective. However, in the method taught in patent document 1, spheroidization of the biocompatible polymer block and MSC is necessary and thus complicated operations are required. In addition, the prior art has many problems to be solved, such as the insufficient amount of the produced paracrine factors in the methods taught in non-patent documents 1 and 2, and the like. Therefore, the present invention aims to provide a means for conveniently and effectively increasing the amount of paracrine factors produced in cells. Also, the present invention aims to provide a novel therapeutic approach utilizing the cells produced by the means and showing promoted production of paracrine factors.

### [Solution to Problem]

The present inventors have conducted intensive studies in an attempt to solve the above-mentioned problems and found that a specific compound can promote production of paracrine factors in cells. Based on such finding, they have conducted further studies and completed the present invention. Therefore, the present invention provides the following.

[1] A medium additive for promoting production of a paracrine factor, comprising a compound represented by the following formula (I): 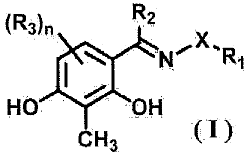 wherein, X is -NHCO-, R₁ is -Y-NH-Z-Ar wherein Y and Z are each a single bond or an alkylene group having 1 - 6 carbon atoms and optionally having substituent(s), and Ar is an aryl group or a heteroaryl group optionally having substituent(s), R₂ is an alkyl group having 1 - 6 carbon atoms and optionally having substituent(s), R₃ is a hydroxyl group, and n is an integer of 0, 1 or 2, or a salt thereof.
[2] The additive of [1], wherein R₂ is a methyl group, an ethyl group, or an isobutyl group,
   n is 0,
   Ar is a phenyl group optionally substituted by a hydroxyl group or a methyl group, and
   Y is a methylene group optionally substituted by a methyl group or an ethyl group.
[3] The additive of [1] or [2], wherein the compound is a compound selected from the group consisting of the following:

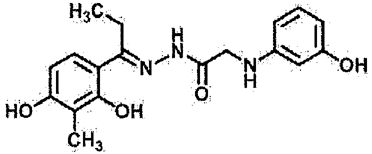

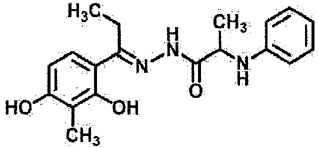

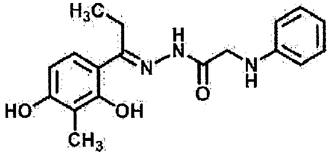

   and 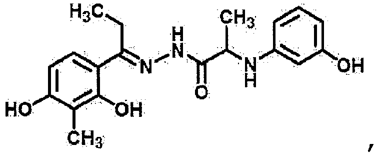 or a salt thereof.
[4] The additive of [1], wherein the compound is a compound represented by: 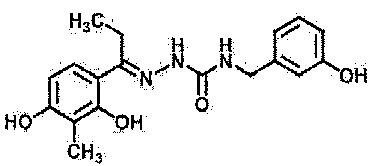 or a salt thereof.
[5] The additive of [1], wherein the compound is a compound represented by:

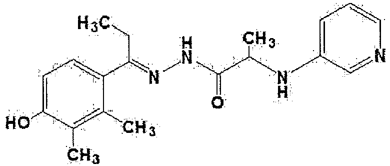

   or 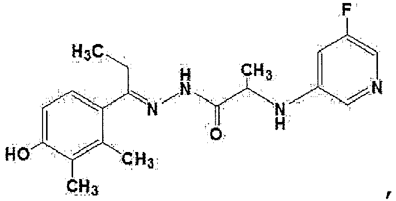 or a salt thereof.
[6] The additive of any of [1] to [5], wherein the paracrine factor is at least one type of protein selected from the group consisting of an anti-inflammatory protein and an angiogenesis promoting protein.
[7] The additive of [6], wherein the anti-inflammatory protein is at least one type of protein selected from the group consisting of TNF-stimulated gene 6 protein (TSG-6) and Stanniocalcin-1 (STC-1).
[8] The additive of [6], wherein the angiogenesis promoting protein is at least one type of protein selected from the group consisting of Angiogenin (ANG), Epidermal Growth Factor (EGF), Monocyte Chemotactic Protein-1 (MCP-1), Epithelial-derived neutrophil-activating peptide 78 (ENA-78), Basic fibroblast growth factor (bFGF), Interleukin-6 (IL-6), Interleukin-8 (IL-8), Vascular endothelial growth factor (VEGF), Vascular endothelial growth factor-D (VEGF-D), Tissue inhibitors of matrix metalloproteinase (TIMP), Platelet-Derived Growth Factor (PDGF), and Transforming growth factor-β (TGF-β).
[9] The additive of any of [1] to [8], wherein the cell is a mesenchymal stem cell.
[10] A cell culture medium comprising the additive of any of [1] to [9].
[11] A method for producing a cell showing promoted production of a paracrine factor, comprising culturing cells in a medium comprising a compound represented by the following formula (I):

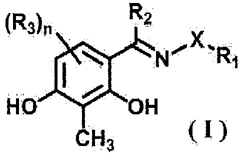

   wherein, X is -NHCO-, R₁ is -Y-NH-Z-Ar wherein Y and Z are each a single bond or an alkylene group having 1 - 6 carbon atoms and optionally having substituent(s), and Ar is an aryl group or a heteroaryl group optionally having substituent(s), R₂ is an alkyl group having 1 - 6 carbon atoms and optionally having substituent(s), R₃ is a hydroxyl group, and n is an integer of 0, 1 or 2,
   or a salt thereof.
[12] The method of [11], wherein R₂ is a methyl group, an ethyl group, or an isobutyl group,
   n is 0,
   Ar is a phenyl group optionally substituted by a hydroxyl group or a methyl group, and
   Y is a methylene group optionally substituted by a methyl group or an ethyl group.
[13] The method of [11] or [12], wherein the compound represented by the formula (I) is a compound selected from the group consisting of the following or a salt thereof:

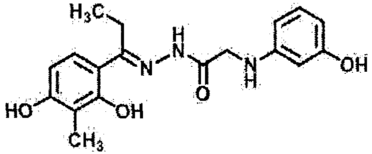

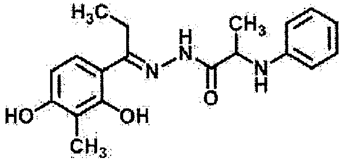

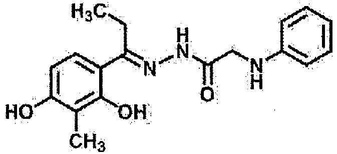

   and 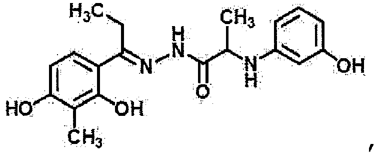 or a salt thereof.
[14] The method of [11], wherein the aforementioned compound is a compound represented by: 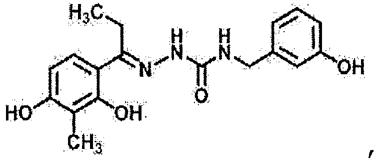 or a salt thereof.
[15] The method of [11], wherein the aforementioned compound is a compound represented by: 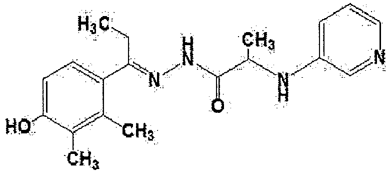 or 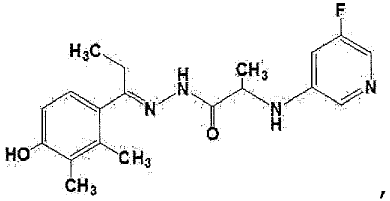 or a salt thereof.
[16] The method of any of [11] to [15], wherein the paracrine factor is at least one type of protein selected from the group consisting of an anti-inflammatory protein and an angiogenesis promoting protein.
[17] The method of [16], wherein the anti-inflammatory protein is at least one type of protein selected from the group consisting of TNF-stimulated gene 6 protein (TSG-6) and Stanniocalcin-1 (STC-1).
[18] The method of [16], wherein the angiogenesis promoting protein is at least one type of protein selected from the group consisting of Angiogenin (ANG), Epidermal Growth Factor (EGF), Monocyte Chemotactic Protein-1 (MCP-1), Epithelial-derived neutrophil-activating peptide 78 (ENA-78), Basic fibroblast growth factor (bFGF), Interleukin-6 (IL-6), Interleukin-8 (IL-8), Vascular endothelial growth factor (VEGF), Vascular endothelial growth factor-D (VEGF-D), Tissue inhibitors of matrix metalloproteinase (TIMP), Platelet-Derived Growth Factor (PDGF), and Transforming growth factor-β (TGF-β).
[19] The method of any of [11] to [18], wherein the cell is a mesenchymal stem cell.
[20] The method of any of [11] to [19], wherein the culture is performed in a three-dimensional culture.
[21] A composition for in vivo transplantation, comprising a cell produced by the method of any of [11] to [20].
[22] A composition for treating an inflammatory disease or ischemic disease, comprising a cell produced by the method of [19] or [20].
[23] The composition of [22], wherein the inflammatory disease is selected from the group consisting of inflammatory bowel disease, ulcerative colitis, Crohn's disease, nephritis, acute nephritis, chronic nephritis, glomerulonephritis, IgA nephropathy, diabetic nephropathy, membranous nephropathy, hydronephrosis, contrast nephropathy, pyelonephritis, renal failure, interstitial nephritis, renal disorder, nephrotic syndrome, hypertensive nephrosclerosis, diabetic glomerulosclerosis, renal calculus, amyloid kidney, renal vein thrombosis, Alport syndrome, hepatitis, cirrhosis, pancreatitis, pneumonia, sinusitis, rhinitis, arthritis, knee osteoarthritis, hand osteoarthritis, hip osteoarthritis, ankle osteoarthritis, hip osteoarthritis, rheumatoid arthritis, periodic fever, aphthous stomatitis, pharyngitis, mucocutaneous lymphnode syndrome, adult onset Still's disease, Behcet's disease, gout, pseudogout, Schnitzler syndrome, chronic relapsing multifocal osteomyelitis, Cryopyrin-associated periodic syndrome, familial cold urticaria, Muckle-Wells syndrome, Chronic infantile neurologic cutaneous, and articular syndrome, Neonatal onset multisystem inflammatory disease, tumor necrosis factor (TNF) receptor associated periodic syndrome, Hyper-IgD syndrome, Blau syndrome, Early-onset sarcoidosis, familial Mediterranean fever, pyogenic arthritis, pyoderma gangrenosum, contusion, Nakajo-Nishimura syndrome, Majeed syndrome, NLRP12-associated periodic syndrome, deficiency of interleukin-1 receptor antagonist deficiency of interleukin-1 receptor antagonist, deficiency of interleukin-36 receptor antagonist, auto-inflammation and phospholipase Cγ2-associated antibody deficiency and immune dysregulation syndrome, HOIL-1 deficiency, SLC29A3 deficiency, CARD14 abnormality, adenosine deaminase 2 deficiency, STING-Associated Vasculopathy with Onset in Infancy, and NLRC4 abnormality.
[24] The composition of [20], wherein the ischemic disease is selected from the group consisting of angina pectoris, myocardial infarction, takotsubo cardiomyopathy, central pulmonary edema, cerebral infarction, ischemic cerebral apoplexy, arteriosclerosis obliterans, and severe lower leg ischemia.
[25] A method for producing a paracrine factor, comprising the following steps:
   (1) a step of culturing a cell in the medium described in [10], and
   (2) a step of recovering a supernatant from the medium used in (1).
[26] The method of [25], wherein the cell is a mesenchymal stem cell.
[27] The method of [25] or [26], wherein the paracrine factor is at least one type of protein selected from the group consisting of an anti-inflammatory protein and an angiogenesis promoting protein.

In one embodiment, moreover, the present invention provides the following:
[1'] A medium additive for promoting production of a paracrine factor, comprising a compound represented by the following formula (I):

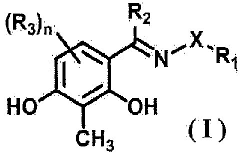

   wherein, X is -NHCO-, R₁ is -Y-NH-Z-Ar wherein Y and Z are each a single bond or an alkylene group having 1 - 6 carbon atoms and optionally having substituent(s), and Ar is an aryl group optionally having substituent(s), R₂ is an alkyl group having 1 - 6 carbon atoms and optionally having substituent(s), R₃ is a hydroxyl group, and n is an integer of 0, 1 or 2, or a salt thereof.
[2'] The agent of [1'], wherein R₂ is a methyl group, an ethyl group, or an isobutyl group,
   n is 0,
   Ar is a phenyl group optionally substituted by a hydroxyl group or a methyl group, and
   Y is a methylene group optionally substituted by a methyl group or an ethyl group.
[3'] The agent of [1'] or [2'], wherein the compound is a compound selected from the group consisting of the following: 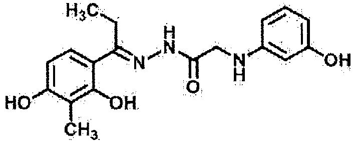 and 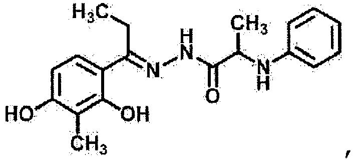 or a salt thereof.
[4'] The agent of [1'], wherein the aforementioned compound is a compound represented by the following: 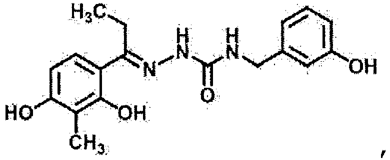 or a salt thereof.
[5'] The additive of any of [1'] to [5'], wherein the paracrine factor is at least one type of protein selected from the group consisting of an anti-inflammatory protein and an angiogenesis promoting protein.
[6'] The additive of [5'], wherein the anti-inflammatory protein is at least one type of protein selected from the group consisting of TNF-stimulated gene 6 protein (TSG-6) and Stanniocalcin-1 (STC-1).
[7'] The additive of [5'], wherein the angiogenesis promoting protein is at least one type of protein selected from the group consisting of Angiogenin (ANG), Epidermal Growth Factor (EGF), Monocyte Chemotactic Protein-1 (MCP-1), Epithelial-derived neutrophil-activating peptide 78 (ENA-78), Basic fibroblast growth factor (bFGF), Interleukin-6 (IL-6), Interleukin-8 (IL-8), Vascular endothelial growth factor (VEGF), Vascular endothelial growth factor-D (VEGF-D), Tissue inhibitors of matrix metalloproteinase (TIMP), Platelet-Derived Growth Factor (PDGF), and Transforming growth factor-β (TGF-β).
[8'] The additive of any of [1'] to [8'], wherein the cell is a mesenchymal stem cell.
[9'] A cell culture medium comprising the additive of any of [1'] to [8'].
[10'] A method for producing a cell showing promoted production of a paracrine factor, comprising culturing cells in a medium comprising a compound represented by the following formula (I):

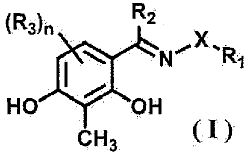

   wherein, X is -NHCO-, R₁ is -Y-NH-Z-Ar wherein Y and Z are each a single bond or an alkylene group having 1 - 6 carbon atoms and optionally having substituent(s), and Ar is an aryl group optionally having substituent(s), R₂ is an alkyl group having 1 - 6 carbon atoms and optionally having substituent(s), R₃ is a hydroxyl group, and n is an integer of 0, 1 or 2, or a salt thereof.
[11'] The method of [10'], wherein R₂ is a methyl group, an ethyl group, or an isobutyl group,
   n is 0,
   Ar is a phenyl group optionally substituted by a hydroxyl group or a methyl group, and
   Y is a methylene group optionally substituted by a methyl group or an ethyl group.
[12'] The method of [10'] or [11'], wherein the compound is a compound selected from the group consisting of the following:

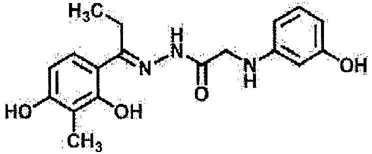

   and 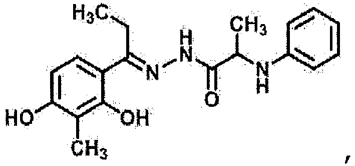 or a salt thereof.
[13'] The method of [10'], wherein the aforementioned compound is a compound represented by: 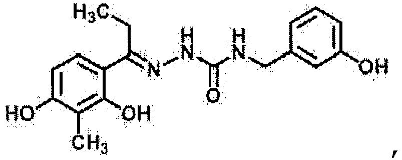 or a salt thereof.
[14'] The method of any of [10'] to [13'], wherein the paracrine factor is at least one type of protein selected from the group consisting of an anti-inflammatory protein and an angiogenesis promoting protein.
[15'] The method of [14'], wherein the anti-inflammatory protein is at least one type of protein selected from the group consisting of TNF-stimulated gene 6 protein (TSG-6) and Stanniocalcin-1 (STC-1).
[16'] The method of [14'], wherein the angiogenesis promoting protein is at least one type of protein selected from the group consisting of Angiogenin (ANG), Epidermal Growth Factor (EGF), Monocyte Chemotactic Protein-1 (MCP-1), Epithelial-derived neutrophil-activating peptide 78 (ENA-78), Basic fibroblast growth factor (bFGF), Interleukin-6 (IL-6), Interleukin-8 (IL-8), Vascular endothelial growth factor (VEGF), Vascular endothelial growth factor-D (VEGF-D), Tissue inhibitors of matrix metalloproteinase (TIMP), Platelet-Derived Growth Factor (PDGF), and Transforming growth factor-β (TGF-β).
[17'] The method of any of [10'] to [16'], wherein the cell is a mesenchymal stem cell.
[18'] The method of any of [10'] to [17'], wherein the culture is performed in a three-dimensional culture.
[19'] A composition for in vivo transplantation, comprising a cell produced by the method of any of [10'] to [18'].
[20'] A composition for treating an inflammatory disease or ischemic disease, comprising a cell produced by the method of [17'] or [18'].
[21'] The composition of [20'], wherein the inflammatory disease is selected from the group consisting of inflammatory bowel disease, ulcerative colitis, Crohn's disease, nephritis, acute nephritis, chronic nephritis, glomerulonephritis, IgA nephropathy, diabetic nephropathy, membranous nephropathy, hydronephrosis, contrast nephropathy, pyelonephritis, renal failure, interstitial nephritis, renal disorder, nephrotic syndrome, hypertensive nephrosclerosis, diabetic glomerulosclerosis, renal calculus, amyloid kidney, renal vein thrombosis, Alport syndrome, hepatitis, cirrhosis, pancreatitis, pneumonia, sinusitis, rhinitis, arthritis, knee osteoarthritis, hand osteoarthritis, hip osteoarthritis, ankle osteoarthritis, hip osteoarthritis, rheumatoid arthritis, periodic fever, aphthous stomatitis, pharyngitis, mucocutaneous lymphnode syndrome, adult onset Still's disease, Behcet's disease, gout, pseudogout, Schnitzler syndrome, chronic relapsing multifocal osteomyelitis, Cryopyrin-associated periodic syndrome, familial cold urticaria, Muckle-Wells syndrome, Chronic infantile neurologic cutaneous, and articular syndrome, Neonatal onset multisystem inflammatory disease, tumor necrosis factor (TNF) receptor associated periodic syndrome, Hyper-IgD syndrome, Blau syndrome, Early-onset sarcoidosis, familial Mediterranean fever, pyogenic arthritis, pyoderma gangrenosum, contusion, Nakajo-Nishimura syndrome, Majeed syndrome, NLRP12-associated periodic syndrome, deficiency of interleukin-1 receptor antagonist deficiency of interleukin-1 receptor antagonist, deficiency of interleukin-36 receptor antagonist, auto-inflammation and phospholipase Cγ2-associated antibody deficiency and immune dysregulation syndrome, HOIL-1 deficiency, SLC29A3 deficiency, CARD14 abnormality, adenosine deaminase 2 deficiency, STING-Associated Vasculopathy with Onset in Infancy, and NLRC4 abnormality.
[22'] The composition of [20'], wherein the ischemic disease is selected from the group consisting of angina pectoris, myocardial infarction, takotsubo cardiomyopathy, central pulmonary edema, cerebral infarction, ischemic cerebral apoplexy, arteriosclerosis obliterans, and severe lower leg ischemia.
[23'] A method for producing a paracrine factor, comprising the following steps:
   (1) a step of culturing a cell in the medium described in [9'], and
   (2) a step of recovering a supernatant from the medium used in (1).
[24'] The method of [23'], wherein the cell is a mesenchymal stem cell.
[25'] The method of [23'] or [24'], wherein the paracrine factor is at least one type of protein selected from the group consisting of an anti-inflammatory protein and an angiogenesis promoting protein.

### [Advantageous Effects of Invention]

According to the present invention, the production of paracrine factors (e.g., anti-inflammatory proteins and angiogenesis promoting proteins) in cells can be promoted very simply and efficiently. The cells produced using the present invention show promoted production and promoted secretion of paracrine factors and thus are considered to be very preferable as cells for transplantation. In addition, the cells produced using the present invention (e.g., mesenchymal stem cell) show remarkably promoted production and secretion of a plurality of anti-inflammatory proteins and angiogenesis promoting proteins among the paracrine factors. Therefore, the cells produced using the present invention (e.g., mesenchymal stem cell) are preferably used for the treatment of anti-inflammatory diseases and ischemic diseases. Furthermore, according to the present invention, pharmaceutically useful paracrine factors derived from cells can be produced and recovered very efficiently.

### [Description of Embodiments]

The terms used in the present specification are defined in the following.

In the present specification, n- means normal, i- means iso, sec- means secondary and tert- means tertiary. In addition, in the present specification, o- means ortho, m-means meta and p- means para.

The "halogen atom" is a fluorine atom, a chlorine atom, a bromine atom, or an iodine atom. The "halogeno group" is fluoro, chloro, bromo, or iodo.

The "alkyl group having 1 - 6 carbon atoms" means a straight chain or branched alkyl group, and specifically, groups such as methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be mentioned. As such alkyl group, lower alkyl group having 1 - 4 carbon atoms, particularly methyl group and ethyl group are preferable.

The "aryl group" is, for example, monocyclic, bicyclic, tricyclic or tetracyclic carbon cyclic group in which at least one ring is aromatic and each ring has 5 to 8 ring atoms. Specifically, phenyl, indenyl, naphthyl, fluorenyl and the like can be mentioned. Particularly, the aryl group may be a C₆₋₁₀ aromatic phenyl, indenyl or naphthyl. The "heteroaryl group" is a cyclic aromatic group having one or more atoms other than carbon within the ring. Specific examples thereof include furanyl group, thiophenyl group, pyridyl group, quinolinyl group, pyrazinyl group, naphthyridil group, benzofuranyl group, benzothiophenyl group, indolyl group, dibenzofuranyl group, dibenzothiophenyl group, carbazolyl group and the like.

The "alkylene group" and "alkylene group having 1 - 6 carbon atoms" mean straight carbon chain. Specifically, groups such as methylene, ethylene, propylene, butylene, pentylene, hexylene and the like can be mentioned.

The "alkyl group", "aryl group" and "alkylene group" may each have a substituent. Examples of such substituent include the following. Examples of the substituent for the "alkyl group" include the following (1) - (40), and examples of the substituent for the "aryl group" and the "alkylene group" include the following (1) - (41).
(1) halogeno group,
(2) hydroxyl group,
(3) cyano group,
(4) nitro group,
(5) carboxyl group,
(6) alkenyl group (C₂₋₁₀ alkenyl group; e.g., vinyl, allyl, propenyl, butenyl, pentenyl, hexenyl, heptenyl, butadienyl, hexatrienyl, and each isomer thereof),
(7) alkynyl group (C₂₋₁₀ alkynyl group; e.g., ethynyl, propynyl, butynyl, pentynyl, hexynyl, and each isomer thereof),
(8) halogenoalkyl group (e.g., monofluoromethyl, difluoromethyl, trifluoromethyl, monofluoroethyl, difluoroethyl, trifluoroethyl, chloromethyl, chloroethyl, dichloroethyl, and each isomer thereof),
(9) cyclic alkyl group (optionally having hetero atom in the ring) (e.g., cyclopropyl, cyclobutyl, cyclopentyl, cyclohexyl, tetrahydrofuranyl, tetrahydropyranyl, aziridinyl, azetidinyl, pyrrolidinyl, piperidinyl, morpholinyl),
(10) aryl group (e.g., phenyl, naphthyl),
(11) heteroaryl group (e.g., pyridyl, pyridazinyl, pyrimidinyl, pyrazinyl, furyl, thiophenyl, pyrrolyl, pyrazolyl, imidazolyl, triazolyl (e.g., 1,2,3-triazolyl, 1,2,4-triazolyl), tetrazolyl, oxazolyl, isoxazolyl, thiazolyl, isothiazolyl, oxadiazolyl (e.g., 1,2,3-oxadiazolyl, 1,2,4-oxadiazolyl, 1,3,4-oxadiazolyl), thiadiazolyl (e.g., 1,2,3-thiadiazolyl, 1,2,4-thiadiazolyl, 1,3,4-thiadiazolyl), benzofuryl, benzothiophenyl, indolyl, isoindolyl, benzoxazolyl, benzothiazolyl, benzimidazolyl, indazolyl, benzisoxazolyl, benzisothiazolyl, benzooxadiazolyl, benzothiadiazolyl, purinyl, quinolinyl, isoquinolinyl, cinnolinyl, phthalazinyl, quinazolinyl, quinoxalinyl, pteridinyl, imidazooxazolyl, imidazothiazolyl, imidazoimidazolyl),
(12) alkoxy group (e.g., methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy),
(13) alkylthio group (e.g., methylthio, ethylthio, n-propylthio, isopropylthio, n-butylthio, isobutylthio, sec-butylthio, tert-butylthio, n-pentylthio, isopentylthio, tert-pentylthio, neopentylthio, 2-pentylthio, 3-pentylthio, n-hexylthio, 2-hexylthio),
(14) alkoxy group (same as the above-mentioned (12)) substituted by aryl group (same as the above-mentioned (10)),
(15) alkylthio group (same as the above-mentioned (13)) substituted by aryl group (same as the above-mentioned (10)),
(16) alkoxy group (same as the above-mentioned (12)) substituted by heteroaryl group (same as the above-mentioned (11)),
(17) alkylthio group (same as the above-mentioned (13)) substituted by heteroaryl group (same as the above-mentioned (11)),
(18) cyclic alkyl (optionally having hetero atom in the ring) oxy group (e.g., cyclopropyloxy, cyclobutyloxy, cyclopentyloxy, cyclohexyloxy, tetrahydrofuranyloxy, tetrahydropyranyloxy, aziridinyloxy, azetidinyloxy, pyrrolidinyloxy, piperidinyloxy, morpholinyloxy),
(19) aryloxy group (e.g., group with aryl group (same as the above-mentioned (10)) bonded to oxygen atom),
(20) heteroaryloxy group (e.g., group with heteroaryl group (same as the above-mentioned (11)) bonded to oxygen atom),
(21) halogenoalkoxy group (e.g., group with halogenoalkyl group (same as the above-mentioned (8)) bonded to oxygen atom),
(22) halogenoalkylthio group (e.g., group with halogenoalkyl group (same as the above-mentioned (8)) bonded to sulfur atom),
(23) alkoxy group (same as the above-mentioned (12)) substituted by hydroxyl group,
(24) alkoxy group (same as the above-mentioned (12)) substituted by alkoxy group (same as the above-mentioned (12)),
(25) amino group,
(26) amino group mono- or di-substituted by alkyl group,
   As used herein, the "alkyl group" is, for example, C₁₋₆ alkyl group. Specifically, methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be mentioned.
(27) carbamoyl group,
(28) carbamoyl group mono- or di-substituted by alkyl group (same as "alkyl group" in the above-mentioned (26)) (e.g., methylcarbamoyl, ethylcarbamoyl, dimethylcarbamoyl, diethylcarbamoyl, ethylmethylcarbamoyl),
(29) sulfamoyl group,
(30) sulfamoyl group mono- or di-substituted by alkyl group (same as "alkyl group" in the above-mentioned (26)) (e.g., methylsulfamoyl, ethylsulfamoyl, dimethylsulfamoyl, diethylsulfamoyl, ethylmethylsulfamoyl),
(31) alkanoyl group (e.g., carbonyl group with hydrogen atom or alkyl group (same as "alkyl group" in the above-mentioned (26)) bonded to carbon atom),
(32) aroyl group (e.g., carbonyl group with aryl group (same as the above-mentioned (10)) bonded to carbon atom),
(33) alkylsulfonylamino group (e.g., sulfonylamino group substituted by alkyl group (same as "alkyl group" in the above-mentioned (26)))
(34) arylsulfonylamino group (e.g., sulfonylamino group substituted by aryl group (same as the above-mentioned (10))),
(35) heteroarylsulfonylamino group (e.g., sulfonylamino group substituted by heteroaryl group (same as the above-mentioned (11))),
(36) acylamino group (e.g., amino group substituted by acyl group),
   As used herein, the "acyl group" is an acyl group having a C₁₋₆ alkyl group, or a C₆₋₁₀ aryl group. As used herein, the "C₁₋₆ alkyl group" is the above-mentioned "alkyl group" having 1 - 6 carbon number, and "C₆₋₁₀ aryl group" is the above-mentioned "aryl group" having 6 - 10 carbon number. Specific examples of the acyl group include acetyl group, propionyl group, butyroyl group, isobutyroyl group, valeroyl group, isovaleroyl group, pivaloyl group, hexanoyl group, acryloyl group, methacryloyl group, crotonoyl group, isocrotonoyl group, benzoyl group, naphthoyl group and the like,
(37) alkoxycarbonylamino group (e.g., carbonylamino group substituted by alkoxy group (same as the above-mentioned (12))),
(38) alkylsulfonyl group (e.g., sulfonyl group substituted by alkyl group (same as "alkyl group" in the above-mentioned (26))),
(39) alkylsulfinyl group (e.g., sulfinyl group substituted by alkyl group (same as "alkyl group" in the above-mentioned (26))),
(40) alkoxycarbonyl group (e.g., methoxycarbonyl group, ethoxycarbonyl group),
(41) alkyl group (C₁₋₆ alkyl group; e.g., methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl etc.) and the like.

When two or more substituents are present, they may be the same or different.

The compound of the formula (I) may be in the form of a salt. Examples of the salt of the aforementioned compound represented by the formula (I) include salts with inorganic acids such as hydrochloric acid and hydrobromic acid, and salts with organic acids such as acetic acid, propionic acid, tartaric acid, fumaric acid, maleic acid, malic acid, oxalic acid, succinic acid, citric acid and benzoic acid. These salts are produced by a method known per se.

The compound represented by the formula (I) may contain geometric isomers of an E-form having an E-steric configuration and Z-form having a Z-steric configuration depending on the type of the substituent. The present invention includes E-form, Z-form or a mixture containing E-form and Z-form in any ratio.

The compound represented by the formula (I) has an optically active substance due to the presence of one or more asymmetric carbon atoms. The compound represented by the formula (I) includes all optically active or racemic compounds.

### [Synthesis of compound represented by the formula (I)]

The compound represented by the formula (I) is preferably obtained by, as shown in the following reaction scheme, using 1 equivalent each of a ketone compound and H₂N-X-R₁ wherein X and R₁ are as defined above, for example, hydrazide compound, semicarbazide compound etc.), and performing the reaction in a solvent such as toluene, 1,4-dioxane, N,N-dimethylformamide, dimethyl sulfoxide and the like at not less than 100°C for 1 hr to 3 days.

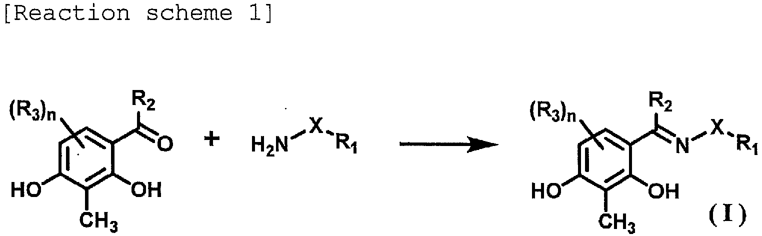

The reaction mixture after completion of the reaction is precipitated by adding distilled water, or when no precipitation occurs, a general post-treatment such as concentration after extraction with an organic solvent is performed to obtain the target specific compound. When purification is necessary, the compound can be separated and purified by any purification method such as recrystallization, column chromatography, thin-layer chromatography, liquid chromatography and the like.

### [Obtainment of optically active form]

Among the compounds synthesized by the aforementioned method, those having optical isomers may have an optically active form (eutomer). An optically active form can be obtained by a method known per se, such as a method by crystallization, a method using an enzymatic reaction, or a method using HPLC (e.g., an optically active support carrier method). Further, the optically active substance can also be prepared using an asymmetric synthesis method or the like.

### 1. Medium additive for promoting production of paracrine factor in cell

The present invention provides a medium additive for promoting production of a paracrine factor in a cell, containing the following specific compound or a salt thereof (hereinafter sometimes to be referred to as "the medium additive of the present invention").

The compound to be contained in the medium additive of the present invention is a compound represented by the formula (I) : 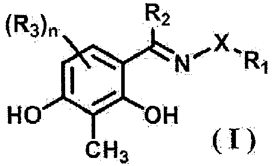
wherein, X is -NHCO-,
R₁ is -Y-NH-Z-Ar wherein Y and Z are each a single bond or an alkylene group having 1 - 6 carbon atoms and optionally having substituent(s), and Ar is an aryl group or heteroaryl group optionally having substituent(s), R₂ is an alkyl group having 1 - 6 carbon atoms and optionally having substituent(s), R₃ is a hydroxyl group, and n is an integer of 0, 1 or 2, or a salt thereof (hereinafter a compound represented by the formula (I) and a salt thereof are sometimes to be generically referred to simply as "the specific compound to be used in the present invention").

In one embodiment, when Y is a single bond, Z is an alkylene group optionally having substituent(s) (more preferably, an alkylene group without a substituent, particularly preferably, a methylene group), Ar is an aryl group optionally having a substituent (more preferably, a halogen atom, a methyl group, a hydroxyl group, or a methoxy group) (more preferably, an aryl group having a hydroxyl group or an aryl group without a substituent, particularly preferably a phenyl group or a phenyl group having a hydroxyl group), or a heteroaryl group optionally having a substituent (more preferably, a halogen atom, a methyl group, a hydroxyl group, or a methoxy group) (more preferably, a pyridyl group optionally having substituent(s), particularly preferably, a pyridyl group without a substituent or a pyridyl group having a fluoro group), R₂ is an alkyl group having 1 - 6 carbon atoms and optionally having substituent(s) (more preferably, an alkyl group having 1 - 6 carbon atoms and free of a substituent, particularly preferably, an ethyl group), and n is an integer of 0, 1 or 2, preferably, n is 0.

When Y is an alkylene group having 1 - 6 carbon atoms and optionally having substituent(s) (more preferably, an alkylene group having 1 - 6 carbon atoms and not having a substituent, particularly preferably a methylene group, an ethylidene group or a propylidene group), Z is a single bond, Ar is an aryl group optionally having substituent(s) (more preferably, an aryl group having a substituent, particularly preferably, a phenyl group having a halogeno group, a methyl group, a hydroxyl group or an ethoxy group, or a naphthyl group), or a heteroaryl group optionally having substituent(s) (more preferably, a heteroaryl group optionally having a halogeno group, particularly preferably, a pyridyl group without a substituent or a pyridyl group having a fluoro group), R₂ is an alkyl group having 1 - 6 carbon atoms and optionally having substituent(s) (more preferably, an alkyl group having 1 - 6 carbon atoms and without a substituent, particularly preferably, a methyl group, an ethyl group, or an isopropyl group), and n is an integer of 0, 1 or 2, preferably, n is 0.

The amount of the specific compound of the present invention which is contained in the medium additive of the present invention is not particularly limited as long as the desired effect of the present invention is obtained. It may be generally 0.01 - 100 wt%, preferably 0.1 - 100 wt%, more preferably 1 - 100 wt%, further preferably 5 - 100 wt%, particularly preferably 10 - 100 wt%.

The medium additive of the present invention can have any shape during provision or preservation. The medium additive of the present invention may be a solid, liquid, gel or the like.

The medium additive of the present invention may be sterilized as necessary. The sterilization method is not particularly limited, and may be appropriately selected according to the shape of the medium additive of the present invention. Examples of the sterilization method include radiation sterilization, ethylene oxide gas sterilization, autoclave sterilization, filter sterilization and the like. When filter sterilization (hereinafter sometimes to be referred to as filtration sterilization) is to be performed, the material of the filter part is not particularly limited and, for example, glass fiber, nylon, PES (polyethersulfone), hydrophilic PVDF (polyvinylidene fluoride), cellulose mixed ester, celluloseacetate, polytetrafluoroethylene and the like can be mentioned. While the size of the pore in the filter is not particularly limited, it is preferably 0.1 µm to 10 µm, more preferably 0.1 µm to 1 µm, most preferably 0.1 µm to 0.5 µm.

In the medium additive of the present invention, to "promote production of paracrine factor in cell" means to increase the ability of the cell to produce paracrine factor and increase the amount of the factor secreted extracellularly. In the present specification, the amount of a paracrine factor is increased by the medium additive of the present invention to at least not less than 120%, at least not less than 130%, at least not less than 140%, at least not less than 150%, at least not less than 160%, at least not less than 170%, at least not less than 180%, at least not less than 190%, at least not less than 200%, at least not less than 300%, at least not less than 400%, at least not less than 500%, at least not less than 600%, at least not less than 700%, at least not less than 800%, at least not less than 900%, at least not less than 1000%, as compared with the amount of a secreted predetermined paracrine factor as a control. For the measurement of the amount of secreted protein, a method known per se such as an ELISA (Enzyme-Linked ImmunoSorbent Assay) method, a flow cytometer method and the like can be used as described in the following Examples.

The paracrine factor whose production is promoted by the medium additive of the present invention includes, but is not limited to, anti-inflammatory proteins such as TSG-6 (TNF-stimulated gene 6 protein), STC-1 (Stanniocalcin-1) and the like, and angiogenesis promoting proteins such as ANG (Angiogenin), EGF (Epidermal Growth Factor), MCP-1 (Monocyte Chemotactic Protein-1), ENA-78 (epithelial-derived neutrophil-activating peptide 78), bFGF (Basic fibroblast growth factor), IL-6 (Interleukin-6), IL-8 (Interleukin-8), VEGF (Vascular endothelial growth factor), VEGF-D (Vascular endothelial growth factor-D), TIMP (Tissue inhibitors of matrix metalloproteinase), PDGF (Platelet-Derived Growth Factor), TGF-β (transforming growth factor-β) and the like. In particular, when the cell is a mesenchymal stem cell, the amounts of produced and secreted TSG-6, STC-1, ANG, and MCP-1 can be significantly increased.

The cell that can be cultured in a medium supplemented with the medium additive of the present invention is not particularly limited as long as the desired effect is obtained, and may be a cell derived from a mammal. Examples of the cell include, but are not limited to, cells such as somatic cells constituting the living body, normal cell line, cancer cell line, progenitor cell, stem cell, cell separated from the living body and applied with artificial genetic modification, cell separated from the living body wherein the nucleus is artificially exchanged and the like. While the derivation of these cells is not particularly limited, the cells derived from mammals such as rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee, human and the like can be mentioned. The tissue or organ from which the cells are derived is not particularly limited as long as the desired effect of the present invention can be obtained. Examples of the aforementioned tissue include tissues such as skin, kidney, spleen, adrenal gland, liver, lung, ovary, pancreas, uterus, stomach, colon, small intestine, large intestine, bladder, prostate, testis, thymus, muscle, connective tissue, bone, cartilages, vascular tissue, blood, heart, eye, brain, nerve tissue and the like. Examples of the organ include organs such as liver, lung, kidney, heart, pancreas, stomach, spleen, small intestine, large intestine, reproductive organ and the like. In the present specification the "stem cells" are cells concurrently having an ability to replicate itself, and an ability to differentiate into other plural lineages. Examples thereof include, but are not limited to, embryonic stem cells (ES cells), embryonic tumor cells, embryonic germ stem cells, induced pluripotent stem cells (iPS cells), neural stem cells, hematopoietic stem cells, mesenchymal stem cells, hepatic stem cells, pancreas stem cells, muscle stem cells, germ stem cells, intestinal stem cells, cancer stem cells, hair follicle stem cells and the like. Examples of the pluripotent stem cells include ES cells, embryonic germ stem cells and iPS cells, from among the aforementioned stem cells. Progenitor cells are cells on the way to differentiate from the aforementioned stem cells into particular somatic cells or reproductive cells. In one preferable embodiment, the cell is human mesenchymal stem cell (hMSC).

A preferable amount of the medium additive of the present invention to be added to a medium is not particularly limited as long as the desired effect of the present invention is achieved. For example, the medium additive of the present invention can be added to a medium such that the concentration of the specific compound to be used in the present invention in the medium is generally 0.001 - 100 µM, preferably 0.01 M - 50 µM, more preferably 0.1 - 30 µM, further preferably 1 - 20 µM, particularly preferably 5 - 10 µM.

A medium to which the medium additive of the present invention can be applied is not particularly limited, and the desired effect of the medium additive of the present invention can be obtained by adding to a commercially available medium. Examples of preferable commercially available medium include, but are not limited to, media such as Dulbecco's Modified Eagle's medium; DMEM, Ham's Nutrient MixtureF12, DMEM/F12 medium, McCoy's 5A medium, Eagle's Minimum Essential medium; EMEM, alpha Modified Eagle's Minimum Essential medium; αMEM, MEM (Minimum Essential medium), RPMI1640 medium, Iscove's Modified Dulbecco's medium; IMDM, MCDB131 medium, Williams' medium E, IPL41 medium, Fischer's medium, StemPro34 (manufactured by Thermo Fisher scientific), X-VIVO 10 (manufactured by Cambrex), X-VIVO 15 (manufactured by Cambrex), HPGM (manufactured by Cambrex), StemSpan H3000 (manufactured by STEMCELL Technologies), StemSpanSFEM (manufactured by STEMCELL Technologies), StemlineII (manufactured by Sigma-Aldrich), QBSF-60 (manufactured by Quality Biological), StemProhESCSFM (manufactured by Thermo Fisher scientific), Essential8 (registered trade mark) medium (manufactured by Gibco), mTeSR1 or 2 medium (manufactured by STEMCELL Technologies), TeSR-E8 medium (manufactured by STEMCELL Technologies), Repro FF or Repro FF2 (manufactured by ReproCELL), Primate ES Cell medium (manufactured by ReproCELL), PSGro hESC/iPSC medium (manufactured by System Biosciences), NutriStem (registered trade mark) medium (manufactured by Biological Industries), StemFit (registered trade mark) medium (manufactured by Ajinomoto Co., Inc.), CSTI-7 medium (manufactured by Cell Science & Technology Institute, Inc.), MesenPRO RS medium (manufactured by Gibco), MF- medium (registered trade mark) mesenchymal stem cell proliferation medium (manufactured by TOYOBO CO., LTD.), Mesenchymal Stem Cell Growth Medium (manufactured by PromoCell GmbH), Mesenchymal Stem Cell Growth Medium 2 (manufactured by PromoCell), MSCGM (manufactured by Lonza), MesenCult XF (manufactured by STEMCELL Technologies), StemPro MSC XF (manufactured by Thermo Fisher scientific), Sf-900II (manufactured by Thermo Fisher scientific), Opti-Pro (manufactured by Thermo Fisher scientific) and the like.

In addition, it is possible to appropriately add further substance such as sodium, potassium, calcium, magnesium, phosphorus, chlorine, various amino acids, various vitamins, antibiotics, serum, fatty acids, sugars, cell growth factors, differentiation inducing factors, cell adhesion factors, antibodies, enzymes, cytokines, hormones, lectins, extracellular matrices, bioactive substances, and the like to the above-mentioned medium. Specific examples include TNF (Tumor Necrosis Factor) α, LPS (Lipopolysaccharide), BMP2 (Bone Morphogenetic Protein 2) and the like.

The cell culture conditions (e.g., temperature, carbon dioxide concentration, culture period etc.) may be those known per se, or may be appropriately modified according to the purpose. For example, the temperature for culturing cells is generally 25 - 39°C, preferably 33 - 39°C (e.g., 37°C). The carbon dioxide concentration is generally 4% - 10% by volume, preferably 4% - 6% by volume, in the atmosphere of culture. The culture period is generally 1 to 35 days, which can be appropriately set according to the purpose of the culture.

When the medium additive of the present invention is added to a three-dimensional cell culture medium, a more remarkable paracrine production promoting effect can be obtained. The three-dimensional cell culture (3D cell culture) in the present specification means, for example, culturing cells in a three-dimensional environment using an embedded culture method, a microcarrier culture method, a sphere culture method, hanging drop culture method, and the like. Embedded culture is a method of cultivating cells by embedding and fixing the cells in a solid or semisolid gel substrate such as Matrigel (registered trade mark), Geltrex (registered trade mark), agar, methylcellulose, collagen, gelatin, fibrin, agarose, alginates and the like. Microcarrier culture method is a method of cultivating cells in a suspended state by proliferating cells in a single layer on the surface of a fine particle slightly heavier than water (hereinafter to be also referred to as a microcarrier), and stirring the fine particles in a culture container such as a flask and the like. Sphere culture is a culture method including forming an aggregate composed of several dozen - several hundred object cells (hereinafter to be also referred to as a sphere or spheroid), and culturing the aggregates with standing or shaking in a medium. As the hanging drop method, for example, a method including spotting a droplet (about 10 - 50 *µ*L in volume) of a cell suspension on the ceiling side such as a lid of a culture vessel, and culturing in an inverted state such that the placed droplet hangs can be mentioned. By culturing in this manner, the cells are minimally influenced by a contact with the flat surface and form a sphere at the bottom of the droplet. Such droplet can also be prepared using a special culture vessel such as GravityPLUS Plate (manufactured by PerkinElmer). As the three-dimensional cell culture (3D cell culture) in the present invention to which the medium additive of the present invention can be applied, a method of culturing cells in a three-dimensional state closer to that in the living body can also be used by dispersing polysaccharides such as hyaluronic acid, deacylated gellan gum, xanthan gum and the like or a derivative of these in a medium to form an atypical three-dimensional network, and maintaining adherent cells suspended in the medium by using the network as a scaffold. At this time, the cells in the three-dimensional cell culture are trapped in the three-dimensional network and do not precipitate. Therefore, the cells can be cultured without a shaking or rotation operation or the like. The three-dimensional cell culture can be performed by a method known per se (e.g., WO 2014/017513).

For the culture of cells, culture vessels generally used for cell culture such as schales, flasks, plastic bags, Teflon (registered trade mark) bags, dishes, petri dishes, dishes for tissue culture, multidishes, microplates, microwell plates, multiplates, multiwell plates, chamber slides, tubes, trays, culture bags, roller bottles and the like can be used for cultivation. In one embodiment, as a culture vessel, one having a surface artificially treated to improve adhesiveness to cells (e.g., coating treatment with extracellular matrix and the like) may be used. Examples of such container include, but are not limited to, coated containers such as Matrigel (registered trade mark), Geltrex (registered trade mark), collagen, gelatin, poly-L-lysine, poly-D-lysine, laminin, fibronectin, vitronectin, tenascin, selectin, hyaluronic acid, fibrin and the like. A coating material for inhibiting adhesion of the cells to culture tools can also be used. Examples of the coating material include, but are not limited to, hydrogel, prevelex (registered trade mark), silicon, poly(2-hydroxymethylmethacrylate), poly(2-methacryloyloxyethylphosphoryl choline) and the like.

The cells can also be cultured by automatically conducting cell seeding, medium exchange, cell image obtainment, and harvasting of cultured cells, under a mechanical control and under a closed environment while controlling pH, temperature, oxygen concentration and the like and using a bioreactor and an automatic incubator capable of high density culture. Examples of the method for supplying a new medium and feeding the required substances to the cells and/or tissues during the culture using such apparatuses include, but are not limited to, fed-batch culture, continuous culture and perfusion culture.

### 2. Cell culture medium

The present invention also provides a cell culture medium containing the medium additive of the present invention (hereinafter sometimes referred to as "the cell culture medium of the present invention").

The cell culture medium of the present invention is prepared by adding the medium additive of the present invention to a medium for cell culture. A medium for cell culture that can be used for preparing the cell culture medium of the present invention includes, but is not limited to, the commercially available medium exemplified in "1. Medium additive for promoting production of paracrine factor in cell". The concentration of the specific compound to be used in the present invention which is contained in the medium additive of the present invention can also be the same as that explained in "1. Medium additive for promoting production of paracrine factor in cell".

In one preferred embodiment, the cell culture medium of the present invention may be a cell culture medium permitting three-dimensional cell culture.

### 3. Method for producing cell showing promoted production of paracrine factor

The present invention also provides a method for producing a cell showing promoted production of a paracrine factor, which includes culturing the cell in a medium containing a specific compound to be used in the present invention (hereinafter sometimes to be referred to as "the production method of the present invention").

In the production method of the present invention, the specific compound to be used in the present invention, the concentration thereof in a medium, usable medium, cell type that can be cultured, culture conditions and the like can be the same as those explained in "1. Medium additive for promoting production of paracrine factor in cell". As the medium to be used in the production method of the present invention, the aforementioned medium of the present invention may also be used.

In one embodiment of the production method of the present invention, cell culture can be performed using a three-dimensional cell culture method. By culturing according to the three-dimensional cell culture method, the production of paracrine factors can be more remarkably promoted. The three-dimensional cell culture method is as described above.

### 4. Composition for in vivo transplantation

The present invention also provides a composition for in vivo transplantation which contains a cell produced by the production method of the present invention (hereinafter sometimes to be referred to as "the composition of the present invention").

The tissue into which the composition of the present invention is transplanted may be determined according to the type of the cells contained therein. For example, when the composition mainly contains mesenchymal stem cells, it can be preferably used for, though not limited to, repair or regeneration of bone tissue, cartilage tissue, adipose tissue, vascular tissue and the like, which are tissues composed of mesenchymal cells.

The content of the cells contained in the composition of the present invention is not particularly limited, and may be appropriately determined according to the application site, application, route, and the like.

The composition of the present invention may contain a pharmaceutically acceptable pharmaceutical additive in addition to the cells produced by the method of the present invention. Examples of the pharmaceutical additive include, but are not limited to, isotonicity agent, buffering agent, pH adjuster, stabilizer, chelating agent, antiseptic and the like.

Examples of the isotonicity agent include sodium chloride, potassium chloride, saccharides, glycerol and the like. Examples of the buffering agent include boric acid, phosphoric acid, acetic acid, citric acid, and corresponding salts thereof (e.g., alkali metal salts and alkaline earth metal salts thereof such as sodium salt, potassium salt, calcium salt, magnesium salt and the like thereof) and the like. Examples of the pH adjuster include inorganic acids such as hydrochloric acid, sulfuric acid, phosphoric acid, polyphosphoric acid, boric acid, borax and the like; organic acids such as acetic acid, propionic acid, oxalic acid, gluconic acid, fumaric acid, lactic acid, citric acid, succinic acid, tartaric acid, malic acid and the like; inorganic bases such as potassium hydroxide, sodium hydroxide and the like; organic bases such as mono ethanolamine, triethanolamine, diisopropanolamine, triisopropanolamine and the like; ammonium acetate, sodium lactate, sodium citrate, potassium carbonate, sodium hydrogen carbonate, sodium carbonate, ammonium bicarbonate, dipotassium phosphate, potassium dihydrogen phosphate, sodium hydrogenphosphate, sodium dihydrogen phosphate, calcium lactate and the like. Examples of the stabilizer include human serum albumin, general L-amino acid, saccharides, cellulose derivative and the like, and these can be used alone or in combination with a surfactant or the like. The above-mentioned L-amino acid may be any of glycine, cysteine, glutamic acid and the like, and is not limited to these. Saccharides may be any of monosaccharides such as glucose, mannose, galactose, fructose and the like, sugar alcohols such as mannitol, inositol, xylitol and the like, disaccharides such as sucrose, maltose, lactose and the like, polysaccharides such as dextran, hydroxypropylstarch, chondroitin sulfuric acid, hyaluronic acid and the like, and derivatives thereof and the like, and are not limited to these. The cellulose derivative may be any of methylcellulose, ethylcellulose, hydroxyethylcellulose, hydroxypropylcellulose, hydroxypropylmethylcellulose, carboxymethylcellulose sodium and the like, and is not limited to these. The chelating agent is exemplified by sodium edetate, citric acid and the like.

The shape of the composition of the present invention is not particularly limited as long as it permits transplantation into a living body. For example, a liquid composed of cells and an appropriate dispersion medium, a sheet in which cells are fixed on an appropriate biocompatible material and the like can be mentioned.

As described above, the cell produced by the method of the present invention and contained in the composition of the present invention shows promoted production and secretion of a paracrine factor. For example, the mesenchymal stem cell produced by the method of the present invention has higher anti-inflammatory effect and higher angiogenesis promoting effect than mesenchymal stem cells not treated with the specific compound used in the present invention. Therefore, the composition of the present invention containing such mesenchymal stem cell as a component is expected to show a higher regeneration effect at the application site by transplanting it into a living body.

### 5. Composition for treatment of inflammatory disease or ischemic disease

The present invention also provides a composition for the treatment of an inflammatory disease or ischemic disease which contains a mesenchymal stem cell produced by the production method of the present invention (hereinafter sometimes to be referred to as "the therapeutic composition of the present invention"). The therapeutic composition of the present invention can treat an inflammatory disease or ischemic disease by administration to a subject suffering from the disease.

While the administration method of the therapeutic composition of the present invention to a subject is not particularly limited as long as a desired effect can be obtained, administration into blood is preferable because it is very simple. When a severely affected area is observed in the subject to be treated, the therapeutic composition of the present invention can also be directly transplanted to the area.

The mesenchymal stem cell to be contained in the therapeutic composition of the present invention may be any mesenchymal stem cell produced by the method of the present invention.

The content of the mesenchymal stem cell to be contained in the therapeutic composition of the present invention is not particularly limited, and can be appropriately determined in consideration of the shape and the like of the composition.

The shape of the therapeutic composition of the present invention is not particularly limited as long as it permits parenteral administration to the subject. For example, it can be in the form of a liquid composed of cells and an appropriate dispersion medium. When the therapeutic composition of the present invention is directly transplanted to a severely affected part, the shape of the therapeutic composition of the present invention may be a sheet in which mesenchymal stem cells are fixed on a biocompatible material.

The amount of the therapeutic composition of the present invention to be administered to a subject is not particularly limited, and may be any amount as long as it is a therapeutically effective amount. The therapeutically effective amount can be appropriately determined in consideration of the degree of the disease, the age and body weight of the subject, the administration method, the administration frequency, the shape of the treatment composition of the present invention, and the like.

The therapeutic composition of the present invention may contain a pharmaceutically acceptable pharmaceutical additive in addition to the mesenchymal stem cells produced by the method of the present invention. Examples of the pharmaceutical additive include, but are not limited to, isotonicity agent, buffering agent, pH adjuster, stabilizer, chelating agent, antiseptic and the like. Specific examples of the pharmaceutical additive are the same as those described in "4. Composition for in vivo transplantation".

Examples of the disease for which the therapeutic composition of the present invention is preferably used include inflammatory diseases and ischemic diseases.

Examples of the inflammatory diseases to which the therapeutic composition of the present invention may be applied include, but are not limited to, inflammatory bowel disease, ulcerative colitis, Crohn's disease, nephritis, acute nephritis, chronic nephritis, glomerulonephritis, IgA nephropathy, diabetic nephropathy, membranous nephropathy, hydronephrosis, contrast nephropathy, pyelonephritis, renal failure, interstitial nephritis, renal disorder, nephrotic syndrome, hypertensive nephrosclerosis, diabetic glomerulosclerosis, renal calculus, amyloid kidney, renal vein thrombosis, Alport syndrome, hepatitis, cirrhosis, pancreatitis, pneumonia, sinusitis, rhinitis, arthritis, knee osteoarthritis, hand osteoarthritis, ankle osteoarthritis, hip osteoarthritis, rheumatoid arthritis, periodic fever with aphthous pharyngitis and adenitis (PFAPA), adult onset Still's disease, Behcet's disease, gout, pseudogout, Schnitzler syndrome, chronic relapsing multifocal osteomyelitis (CRMO), Cryopyrin-associated periodic syndrome (CAPS), familial cold urticaria, Muckle-Wells syndrome, Chronic infantile neurologic cutaneous, and articular syndrome (CINCA syndrome)/Neonatal onset multisystem inflammatory disease (NOMID), TNF (tumor necrosis factor) receptor-associated periodic syndrome (TRAPS), Hyper-IgD syndrome (Mevalonate Kinase Deficiency), Blau syndrome/Early-onset sarcoidosis, familial Mediterranean fever, PAPA (Pyogenic Arthritis, Pyoderma gangrenosum and Acne) syndrome, Nakajo-Nishimura syndrome, Majeed syndrome, NLRP12-associated periodic syndrome (NAPS12), deficiency of interleukin-1 receptor antagonist (DIRA), deficiency of interleukin-36 receptor antagonist (DITRA), phospholipase Cγ2-associated antibody deficiency and immune dysregulation syndrome (PLAID), HOIL-1 deficiency, SLC29A3 deficiency, CARD14 abnormality, ADA2 (adenosine deaminase 2) deficiency, STING-Associated Vasculopathy with Onset in Infancy (SAVI) and NLRC4 abnormality and the like.

Examples of the ischemic disease to which the therapeutic composition of the present invention is applied include, but are not limited to, angina pectoris, myocardial infarction, takotsubo cardiomyopathy, central pulmonary edema, cerebral infarction, ischemic cerebral apoplexy, arteriosclerosis obliterans, severe lower leg ischemia and the like.

### 6. Composition for treatment of inflammatory disease or ischemic disease

The present invention is also directed to a method for producing a paracrine factor, including the following steps (hereinafter sometimes referred to as "the production method of paracrine factor of the present invention"):
(1) a step of culturing a cell in the medium of the present invention, and
(2) a step of harvesting a supernatant from the medium used in (1).

The cell used in step (1) of the production method of paracrine factor of the present invention may be any that can produce and secrete a paracrine factor, and may be, for example, the cell explained in "1. Medium additive for promoting production of paracrine factor in cell". In one preferred embodiment, the cell is a mesenchymal stem cell.

The conditions for cell culture are not particularly limited, and may be any as long as the cells to be cultured are maintained and proliferated. Preferable cell culture conditions can be easily determined by those skilled in the art by using known methods. In one preferred embodiment, the cells are cultured by three-dimensional culture.

The cells can also be cultured by automatically conducting cell seeding, medium exchange, cell image obtainment, and harvesting of cultured cells, under a mechanical control and under a closed environment while controlling pH, temperature, oxygen concentration and the like and using a bioreactor and an automatic incubator capable of high density culture. Examples of the method for supplying a new medium and feeding the required substances to the cells during the culture using such apparatuses include, but are not limited to, fed-batch culture, continuous culture and perfusion culture.

By culturing cells using the medium of the present invention, the cells efficiently produce a paracrine factor, and as a result, a large amount of the produced paracrine factor is secreted into the medium. Therefore, by collecting the supernatant of the medium, the paracrine factor produced and secreted in a large amount can be recovered.

The method for recovering the supernatant in step (2) of the production method of paracrine factor of the present invention is not particularly limited, and a known supernatant recovery method may be used. For example, the culture medium containing the cultured cells is subjected to centrifugation and, after precipitation of the cells, the supernatant can be collected using a pipette or the like; however, the method is not limited thereto.

A larger amount of the paracrine factor can also be recovered by subjecting the precipitated cells to the culture of step (1) again and repeating the production method of paracrine factor of the present invention.

The present invention is explained in more specifically in the following by referring to Examples; however, the present invention is not limited in any way by the following Examples.

### [Example]

### [Synthetic Example of compounds]

In the present specification, a ketone of 2',4'-dihydroxy-3'-methylpropiophenone (sometimes to be also referred to as "k-1") can be synthesized by a method known per se (Sum TH et al., Tetrahedron. 2015 Jul 1; 71(26-27): 4557-4564.). In addition, a hydrazine of 2-(phenylamino)acetohydrazide (sometimes to be also referred to as "H-1") can also be synthesized by a method known per se (Samal RP et al., Chem Biol Drug Des. 2013 Jun; 81(6):715-29.).

### [Synthetic Example 1] Synthesis method of k-1:B-1

Methyl 2-bromopropionate (109) (500 mg, 3.0 mmol) was dissolved in DMSO (6 mL), aniline (0.36 mL, 3.9 mmol), potassium carbonate (0.54 g, 3.9 mmol) were added and the mixture was stirred at room temperature for 21 hr. Ethyl acetate (30 mL), water (50 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel chromatography (silica gel 30 g, ethyl acetate/hexane=2/98 - 15/85) to give N-phenylalanine methyl ester (110) (343 mg, 1.91 mmol, yield 64%) as a light yellow liquid.

110 (340 mg, 1.9 mmol) obtained as mentioned above was dissolved in methanol (3.8 mL), hydrazine monohydrate (0.18 mL, 3.8 mmol) was added, and the mixture was stirred at 60°C for 24 hr. Hydrazine monohydrate (0.36 mL, 7.4 mmol) was added and the mixture was further stirred at 60°C for 17 hr. The reaction solution was concentrated under reduced pressure. The obtained residue was purified by moderate-pressure silica gel chromatography (aminesilica gel 10 g, ethyl acetate/methylene chloride=0/100 - 20/80) to give 2-(phenylamino)propanehydrazide (111) (321 mg, 1.79 mmol, yield 94%) as a colorless solid.

111 (168 mg, 0.937 mmol) obtained as mentioned above, and 2',4'-dihydroxy-3'-methylpropiophenone (k-1) (130 mg, 0.72 mmol) were dissolved in DMSO (1.4 mL), and the mixture was stirred at 100°C for 19 hr. The mixture was allowed to cool, distilled water (15 mL) was added, and the precipitated solid was collected by filtration, and dried. The obtained yellow solid was suspension-washed with methylene chloride, and dried under reduced pressure to give k-1:B-1 (173 mg, 0.507 mmol, yield 70%) as a light yellow solid.

### [Synthetic Example 2] Synthesis method of k-1:D-1

3-Benzyloxyaniline (129) (2.44 g, 12.2 mmol) was dissolved in DMF (24 mL), sodium acetate (1.10 g, 13.5 mmol), ethyl bromoacetate (128) (1.49 mL, 13.5 mmol) were added and the mixture was stirred at room temperature for 4 hr. Water (300 mL), ethyl acetate (150 mL) were added and the mixture was partitioned. The organic layer was washed with saturated brine (100 mL), dried over anhydrous sodium sulfate, filtered, and concentrated under reduced pressure. The obtained residue was purified by moderate-pressure silica gel column chromatography (silica gel 50 g, ethyl acetate/hexane=2/98 - 10/90) to give N-(3-benzyloxyphenyl)glycine ethyl ester (130) (2.82 g, 9.88 mmol, yield 81%) as a light yellow solid.

130 (1.0 g, 3.5 mmol) obtained as mentioned above was suspended in methanol (18 mL), 10% Pd/C (0.1 g) was added and the mixture was stirred under a hydrogen atmosphere at room temperature for 5 hr. The reaction solution was filtered through celite, and the filtrate was concentrated under reduced pressure. The obtained residue was purified by moderate-pressure silica gel column chromatography (silica gel 30 g, ethyl acetate/hexane=3/97 - 35/65) to give N-(3-hydroxyphenyl)glycine ethyl ester (131) (295 mg, 1.51 mmol, yield 43%) as a light yellow liquid.

131 (0.29 g, 1.5 mmol) obtained as mentioned above was dissolved in ethanol (3.5 mL), hydrazine monohydrate (0.32 mL, 6.5 mmol) was added, and the mixture was stirred at 60°C for 18 hr. The reaction solution was concentrated under reduced pressure, and the obtained residue was purified by moderate-pressure silica gel chromatography (aminesilica gel 10 g, methanol/methylene chloride=1/99 - 10/90). The obtained solid was suspension-washed with IPE, and dried under reduced pressure to give 2-[(3-hydroxyphenyl)amino]acetohydrazide (132) (239 mg, 1.32 mmol, yield 88%) as a light yellow solid.

132 (130 mg, 0.72 mmol) obtained as mentioned above, and 2',4'-dihydroxy-3'-methylpropiophenone (k-1) (100 mg, 0.55 mmol) were dissolved in DMSO (1.1 mL), and the mixture was stirred at 100°C for 21 hr. The mixture was allowed to cool, and the reaction solution was directly purified by moderate-pressure silica gel column chromatography (silica gel 10 g, ethyl acetate/methylene chloride=5/95 - 50/50). The obtained solid was suspension-washed with water and IPE, and dried under reduced pressure to give k-1:D-1 (95.9 mg, 0.279 mmol, yield 51%) as a colorless solid.

### [Synthetic Example 3] Synthesis method of k-1:J-1

To ice-cooled THF (10 mL) were successively added hydrogenated aluminum (1.0 g, 26 mmol), 3-cyanophenol (148) (0.63 g, 5.3 mmol), and the mixture was stirred at room temperature for 1.5 hr, and at 60°C for 3.5 hr. The mixture was allowed to cool, hydrogenated aluminum (1.0 g, 26 mmol), THF (10 mL) were added and the mixture was further stirred at 60°C for 16 hr. The reaction solution was ice-cooled, water (1.5 mL), 15% aqueous sodium hydroxide solution (1.5 mL), water (4.5 mL) were successively added and the mixture was stirred at room temperature for 3 hr. The suspended solution was filtered through celite, and the filtrate was concentrated under reduced pressure. The obtained residue was purified by moderate-pressure silica gel column chromatography (aminesilica gel 10 g, methanol/methylene chloride=0/100 - 8/92). The obtained solid was washed with IPE, and dried under reduced pressure to give 3-(aminomethyl)phenol (149) (477 mg, 3.87 mmol, yield 73%) as a colorless solid.

149 (200 mg, 1.6 mmol) obtained as mentioned above was dissolved in methylene chloride (2 mL), water (2 mL), sodium hydrogen carbonate (0.27 g, 3.2 mmol) was added and phenyl chloroformate (136) (0.22 mL, 1.7 mmol) was slowly added dropwise under ice-cooling. The mixture was stirred at room temperature for 20 hr, ethyl acetate (20 mL), water (20 mL) were added and the mixture was partitioned. The organic layer was washed with saturated brine (20 mL), dried over anhydrous magnesium sulfate, filtered, and concentrated under reduced pressure. The obtained residue was purified by moderate-pressure silica gel column chromatography (silica gel 10 g, ethyl acetate/hexane=5/95 - 35/65) to give phenyl (3-hydroxybenzyl)carbamate (150)(369 mg, 1.52 mmol, yield 95%) as a colorless liquid.

150 (365 mg, 1.50 mmol) obtained as mentioned above was suspended in acetonitrile (3.8 mL), hydrazine monohydrate (0.18 mL, 3.8 mmol) was added and the mixture was stirred at room temperature for 2.5 hr. Hydrazine monohydrate (0.18 mL, 3.8 mmol) was added and the mixture was further stirred at 55°C for 20 hr. The reaction solution was concentrated under reduced pressure, and the obtained solid was suspension-washed with IPE/methylene chloride (3/1), and dried under reduced pressure to give N-(3-hydroxybenzyl)hydrazine carboxyamide (151) (233 mg, 1.29 mmol, yield 86%) as a colorless solid.

151 (130 mg, 0.72 mmol) obtained as mentioned above, and 2',4'-dihydroxy-3'-methylpropiophenone (k-1) (100 mg, 0.55 mmol) were dissolved in DMSO (1.1 mL), and the mixture was stirred at 100°C for 15 hr. The mixture was allowed to cool, and the reaction solution was directly purified by moderate-pressure silica gel column chromatography (silica gel 10 g, ethyl acetate/methylene chloride=10/90 - 55/45). To the obtained purified product was added water and the precipitated solid was collected by filtration and dried under reduced pressure to give k-1:J-1 (102 mg, 0.297 mmol, yield 54%) as a colorless solid.

### [Synthetic Example 4] Synthesis method of k-1:H-1

k-1 (100 mg, 0.555 mmol), H-1 (110 mg, 0.666 mmol) were dissolved in DMSO (1.1 mL), and the mixture was stirred at 100°C for 14 hr. The mixture was allowed to cool, distilled water (11 mL) was added, and the mixture was stirred again at 100°C and filtered while hot to give k-1:H-1 (70.1 mg, 0.214 mmol, yield 39%) as a light yellow solid.

### [Synthetic Example 5] Synthesis method of GA-002A

k-1:B-1 (racemate) synthesized in the aforementioned Synthetic Example 1 was optically resolved by supercritical fluid chromatography (SFC) manufactured by Waters according to the following conditions to obtain the following two enantiomers. The specific optical rotation of the both enantiomers was measured using an optical rotation meter P-1020 (manufactured by JASCO Corporation). The steric configuration (R form, S form) of the enantiomers was determined by X-ray crystal structure analysis.
compound GA-002A (levorotatory enantiomer of k-1:B-1); retention time 11.95 min, fraction 475.1 mg, optical purity 99.9ee%, purity 99.0%, specific optical rotation [α]²²_{D} -10.5 (c=0.1019, ethanol), steric configuration R form

### <fractionation conditions>

column: CHIRALPAK IA <manufactured by Daicel Corporation, 20*250 mm, 5 µm>, weak solvent: CO₂ (70%), strong solvent: MeOH (30%), column temperature: 40°C, total charge: 1 g, flow rate: 15 mL/min

### [Synthetic Example 6] Synthesis method of GA-005A

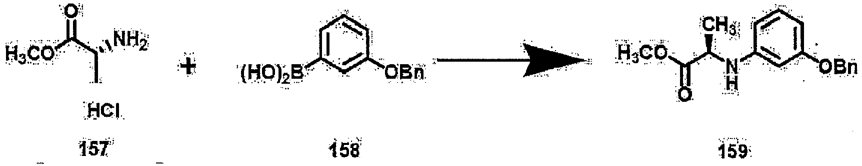

wherein Bn is a benzyl group.

D-alanine methyl ester hydrochloride (157) (1.40 g, 10.0 mmol), 3-benzyloxyphenylboronic acid (158) (3.43 g, 15.1 mmol), copper acetate (II) monohydrate (3.00 g, 15.1 mmol), and molecular sieve 4A (MS4A; 20 g) were dissolved in methylene chloride (200 mL), triethylamine (4.17 mL, 30.1 mmol) was added and the mixture was stirred under oxygen atmosphere at room temperature for 23 hr. The reaction solution was filtered through celite, and the filtrate was concentrated to half under reduced pressure. Saturated aqueous sodium hydrogen carbonate solution (50 mL) was added and the mixture was partitioned. The organic layer was washed with saturated brine (20 mL), dried over anhydrous sodium sulfate, filtered, and concentrated under reduced pressure. The obtained residue was purified by moderate-pressure silica gel chromatography (silica gel 30 g, ethyl acetate/hexane=1/99 - 10/90) to give 159 (595 mg, 2.09 mmol, yield 21%) as a yellow liquid.

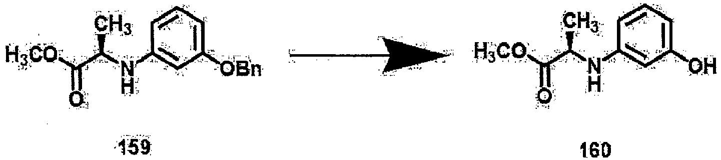

wherein Bn is a benzyl group.

159 (595 mg, 2.09 mmol) obtained as mentioned above was dissolved in methanol (21 mL), palladium carbon ethylenediamine complex (241 mg) was added and the mixture was stirred under hydrogen atmosphere at room temperature for 3.5 hr. Palladium carbon was filtered off and the filtrate was concentrated under reduced pressure. The obtained residue was purified by moderate-pressure silica gel column chromatography (silica gel 30 g, ethyl acetate/hexane=10/90 - 30/70) to give 160 (413 mg, 2.12 mmol, quant.) as a yellow liquid.

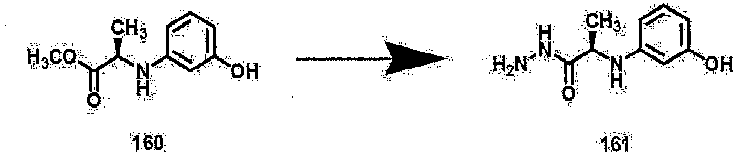

160 (413 mg, 2.12 mmol) obtained as mentioned above was dissolved in methanol (4.2 mL), hydrazine monohydrate (1.0 mL, 21 mmol) was added and the mixture was stirred at 60°C for 3.5 hr. The reaction solution was concentrated under reduced pressure, azeotropically distilled with toluene, and the obtained residue was purified by moderate-pressure silica gel column chromatography (silica gel 10 g, methanol/methylene chloride=1/99 - 10/90) to give 161 (391 mg, 2.00 mmol, yield 95%) as a brown amorphous form.

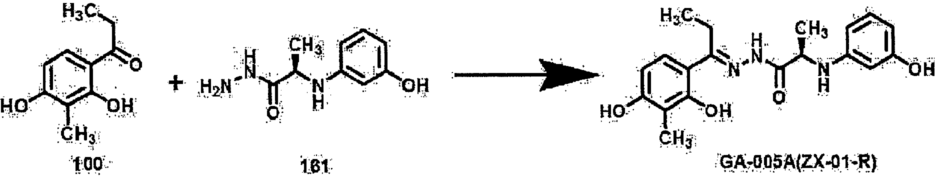

161 (72 mg, 0.37 mmol) obtained as mentioned above and 2',4'-dihydroxy-3'-methylpropiophenone (100) (60 mg, 0.33 mmol) were dissolved in DMSO (0.67 mL), and the mixture was stirred at 100°C for 3 days. The reaction solution was directly purified by moderate-pressure silica gel column chromatography (silica gel 10 g, ethyl acetate/methylene chloride=5/95 - 50/50), and the obtained purified product was successively suspended and washed with distilled water, isopropyl alcohol (IPA)/hexane, distilled water to give GA-005A (ZX-01-R (40.5 mg, 0.113 mmol, yield 34%)) as a pale-brown solid. The specific optical rotation was measured by a polarimeter P-1020 (manufactured by JASCO Corporation).

1H-NMR(270MHz); ; δ13.65(s, 1H), 10.75(s, 1H), 9.70(s, 1H), 8.97(s, 1H), 7.25(d, J=8.1Hz, 1H), 6.83(t, J=8.1Hz, 1H), 6.39(d, J=8.1Hz, 1H), 6.15-5.95(m, 3H), 5.81(d, J=8.1Hz, 1H), 4.18(m, 1H), 2.78(q, J=8.1Hz, 2H), 1.95(s, 3H), 1.37(d, J=8.1Hz, 3H), 1.03(t, J=8.1Hz, 3H).
compound GA-005A; optical purity 99.9ee%, purity 99.2%, specific optical rotation [α]22D-11.4 (c=0.0264, ethanol), steric configuration R form

### [Synthetic Example 7] Synthesis method of A-004

By a method according to Synthetic Example 1, A-004 (81.6 mg) was synthesized from 2-(pyridine-3-amino)propanehydrazide (135 mg) and 2',4'-dihydroxy-3'-methylpropiophenone (135 mg).

### [Synthetic Example 8] Synthesis method of A-004A

Using supercritical fluid chromatography (SFC) manufactured by Waters, A-004 was optically resolved to give 6.6 mg of A-004A.
[optical resolution conditions]
column; CHIRALPAK IA-3 (20x250mm, 5 µm; manufactured by Daicel) mobile phase; CO2:MeOH=60:40 (volume ratio)
column temperature; 40°C
flow rate; 15 mL/min

Under the above-mentioned conditions, the retention time of A-004A (optical purity 99.9ee%) was 8.14 min.

### [Synthetic Example 9] Synthesis method of A-007

By a method according to Synthetic Example 1, A-007 (94.4 mg) was synthesized from 2-(pyridine-3-amino-5-fluoro)propanehydrazide (143 mg) and 2',4'-dihydroxy-3'-methylpropiophenone (100 mg).

A compound represented by the formula (I) can be synthesized according to the aforementioned Synthetic Examples 1 - 9. The compounds represented by the formula (I) and synthesized by the aforementioned Synthetic Examples are shown in the first Table to the third Table; however, the specific compounds used in the present invention are not limited to these alone.

In the table, Me is methyl, and similarly, Et is ethyl, n-Pr and Pr-n are normal propyl, and i-Bu is isobutyl. R₅ is a substituent of an aryl group and is the same as above. In (R₃)ₙ and (R₅)ₘ, "-" means unsubstituted, and the numbers indicated in the structural formulas show the substitutable position of (R₃)ₙ or (R₅)ₘ. n is an integer of 0, 1 or 2, and m is an integer of 0, 1, 2, 3, 4 or 5. When R₅ is present in plurality, they may be the same or different.

### [First Table]

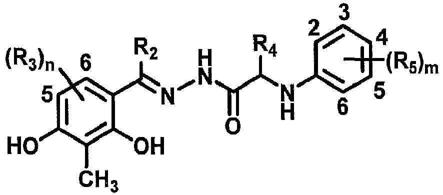

**[Table 1]**

| compound No. | R2 | (R3)n | R4 | (R5)m |
|---|---|---|---|---|
| k-1:B-1 | Et | - | Me | - |
| k-1:D-1 | Et | - | H | 3-OH |
| k-1:H-1 | Et | - | H | - |
| GA-005A | Et | - | Me | 3-OH |

### [Second Table]

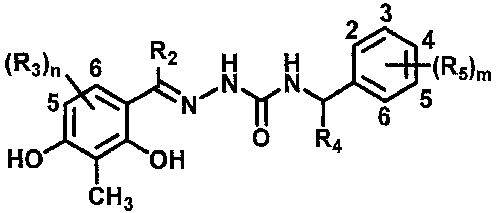

**[Table 2]**

| compound No. | R2 | (R3)n | R4 | (R5)m |
|---|---|---|---|---|
| k-1:J-1 | Et | - | H | 3-OH |

### [Third Table]

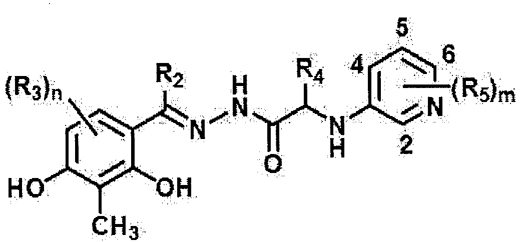

**[Table 3]**

| compound No. | R2 | (R3)n | R4 | (R5)m |
|---|---|---|---|---|
| A-004 | Et | - | Me | - |
| A-007 | Et | - | Me | 5-F |

Among the compounds represented by the formula (I), ¹H-NMR data of the compounds described in the first Table to the third Table are shown below (NMR data of GA-005A are omitted below since they are indicated above).

The proton nuclear magnetic resonance chemical shift value was measured in deuterodimethyl sulfoxide at 270MHz or 400MHz with the value of deuterodimethyl sulfoxide as 2.49 ppm. The symbols in the Table mean the following. s: singlet, brs: broad singlet, d: doublet, dd: double doublet, t: triplet, q: quartet, m: multiplet.

### k-1:B-1; 400MHz

δ10.82(s, 1H), 9.71(s, 1H), 7.25(d, J=8.0Hz, 1H), 7.07(t, J=8.0Hz, 2H), 6.63(d, J=8.0Hz, 2H), 6.56(t, J=8.0Hz, 1H), 6.39(d, J=8.0Hz, 1H), 5.93(d, J=12Hz, 1H), 4.28(m, 1H), 2.80(q, J=8.0Hz, 2H), 1.95(s, 3H), 1.40(d, J=8.0Hz, 3H)1.03(t, J=8.0Hz, 3H). (one signal of NH was not observed)

### k-1:D-1; 270MHz

δ 13.66(s, 1H), 10.76(s, 1H), 9.70(s, 1H), 8.98(s, 1H), 7.25(d, J=10.8Hz, 1H), 6.86(t, J=10.8Hz, 1H), 6.40(d, J=8.1Hz, 1H), 6.20-5.95(m, 3H), 5.87(t, J=5.4Hz, NH), 3.88(d, J=5.4Hz, 2H), 2.78(q, J=8.0Hz, 2H), 1.97(s, 3H), 1.05(t, J=8.1Hz, 3H).

### k-1:J-1; 270MHz

δ13.45(br, 1H), 9.57(s, 1H), 9.53(s, 1H), 9.36(s, 1H), 7.17(d, J=8.1Hz, 1H), 7.11(d, J=8.1Hz, 1H), 6.80-6.70(m, 3H), 6.64(br, NH), 6.37(d, J=8.1Hz, 1H), 4.25(d, J=5.4Hz, 2H), 2.65(q, J=8.1Hz, 2H), 1.97(s, 3H), 1.08(t, J=8.1Hz, 3H).

### k-1:H-1; 270MHz

δ13.98(s, 1H), 11.13(s, 1H), 10.01(s, 1H), 7.58(d, J=8.1Hz, 1H), 7.41(t, J=8.1Hz, 2H), 6.95(d, J=8.1Hz, 2H), 6.89(t, J=8.1Hz, 1H), 6.72(d, J=8.1Hz, 1H), 6.29(t, J=8.1Hz, NH), 4.27(d, J=8.1Hz, 2H), 3.11(q, J=8.1Hz, 2H), 2.29(s, 3H), 1.37(t, J=8.1Hz, 3H).

### A-004; 270MHz

δ13.63(s, 1H), 10.91(s, 1H), 9.71(s, 1H), 8.02(d, J=2.7Hz, 1H), 7.79(d, J=5.4Hz, 1H), 7.26(d, J=8.1Hz, 1H), 7.08(t, J=8.1Hz, 1H), 6.94(d, J=8.1Hz, 1H), 6.40(d, J=8.1Hz, 1H), 6.23(d, J=8.1Hz, 1H), 4.33(m, J=8.1Hz, 1H), 2.82(q, J=8.1Hz, 2H), 1.95(s, 3H), 1.42(d, J=8.1Hz, 3H), 1.05(t, J=8.1Hz, 3H).

### A-007; 270MHz

δ13.61(s, 1H), 10.97(s, 1H), 9.73(s, 1H), 7.89(brs, 1H), 7.71(brs, 1H), 7.26(d,J=8.1Hz, 1H), 6.98(d, J=8.1Hz, 1H), 6.79(d, J=13.5Hz, 1H), 6.69(d, J=8.1Hz, 1H), 6.38(d, J=8.1Hz, 1H), 4.34(m, 1H), 2.82(q, J=8.1Hz, 2H), 1.93(s, 3H), 1.40(d, J=8.1Hz, 3H), 1.05(t, J=8.1Hz, 3H).

### [Example 1] paracrine factor release amount test 1

### (recovery of culture supernatant)

Human bone marrow-derived mesenchymal stem cells (hMSC, manufactured by PromoCell) were precultured by a single layer culture method (2D) using Mesenchymal Stem Cell Growth Medium 2 (manufactured by PromoCell). hMSCs were suspended in the same medium. In three-dimensional culture method (3D), hMSCs were seeded at 5×10⁵ cells/2 mL/well on a cell-non-adhesive 6-well EZSPHERE plate (manufactured by AGC TECHNO GLASS, #4810-900) having a depression on the bottom surface. In 2D, hMSCs were seeded on a 6-well flat bottom plate (manufactured by Corning Incorporated, #3516) at 5×10⁵ cells/2 mL/well. Continuously, compounds k-1:B-1, k-1:D-1, k-1:J-1 dissolved in DMSO were added at 2 µL/well to a final concentration of 5 µM, and the mixture was cultured in an incubator at 37°C, 5% CO₂ for 48 hr. As a control, DMSO was added at a final concentration of 0.05%. After 48 hr, the medium was exchanged with a medium not containing the compound, and the cells were cultured in an incubator at 37°C, 5% CO₂. After 48 hr, the culture supernatant was recovered. The number of cells at the time of recovery of the culture supernatant was measured by dispersing the cells in single cells using Detach Kit (manufactured by PromoCell), suspending a part thereof in Trypan Blue (manufactured by Wako Pure Chemical Industries, Ltd.) and counting the number of viable cells using TC-20 (manufactured by BIO-RAD).

### (quantification using ELISA)

Angiogenin (ANG), a cytokine which is one kind of paracrine factor contained in the culture supernatant, was quantified using a plate reader. For the quantification, Human Angiogenin ELISA Kit (manufactured by Abcam, #ab99970) was used.

The concentration of ANG in the culture supernatant was normalized by the cell count, and the relative values for the control group cultured in 2D are shown in Table 4. As a result, the secretion amount increased in both 2D culture and 3D culture by adding compounds k-1:B-1, k-1:D-1, and compound k-1:J-1. Therefrom it was clarified that the secretion of the paracrine factor can be promoted by adding the compounds k-1:B-1, k-1:D-1, and compound k-1:J-1 to the mesenchymal stem cells.

**[Table 4]**

| (relative value of secretion amount (pg) per 10000 cells) | | |
|---|---|---|
| culture method | compound | ANG |
| 2D | DMSO | 1.00 |
| | k-1:B-1 | 1.34 |
| 3D | DMSO | 1.08 |
| | k-1:B-1 | 1.38 |
| | k-1:D-1 | 1.50 |
| | k-1:J-1 | 1.38 |

### [Example 2] paracrine factor release amount test 2

### (recovery of culture supernatant)

Human bone marrow-derived mesenchymal stem cells (hMSC, manufactured by PromoCell) were precultured by a single layer culture method (2D) using Mesenchymal Stem Cell Growth Medium 2 (manufactured by PromoCell). hMSCs were suspended in the same medium. In three-dimensional culture method (3D), hMSCs were seeded at 5×10⁵ cells/2 mL/well on a cell-non-adhesive 6-well EZSPHERE plate (manufactured by AGC TECHNO GLASS, #4810-900) having a depression on the bottom surface. In 2D, hMSCs were seeded on a 6-well flat bottom plate (manufactured by Corning Incorporated, #3516) at 5×10⁵ cells/2 mL/well. Continuously, compound k-1:B-1 dissolved in DMSO was added at 2 µL/well to a final concentration of 5 µM, and the mixture was cultured in an incubator at 37°C, 5% CO₂ for 48 hr. As a control, DMSO was added at a final concentration of 0.05%. After 48 hr, the medium was exchanged with a medium not containing the compound, and the cells were cultured in an incubator at 37°C, 5% CO₂. After 48 hr, the culture supernatant was recovered. The number of cells at the time of recovery of the culture supernatant was measured by dispersing the cells in single cells using Detach Kit (manufactured by PromoCell), suspending a part thereof in Trypan Blue (manufactured by Wako Pure Chemical Industries, Ltd.) and counting the number of viable cells using TC-20 (manufactured by BIO-RAD).

### (quantification using ELISA)

Stanniocalcin-1 (STC-1), a cytokine which is one kind of paracrine factor contained in the culture supernatant, was quantified using a plate reader. For the quantification, Human Stanniocalcin ELISA Kit (manufactured by Abcam, #ab213829) was used.

The concentration of STC-1 in the culture supernatant was normalized by the cell count, and the relative values for the control group cultured in 2D are shown in Table 5. As a result, the secretion amount increased in 3D culture as compared to 2D culture. In addition, the secretion amount increased in both 2D culture and 3D culture by adding compound k-1:B-1, and the secretion amount of STC-1 increased 8.95-fold in the 3D-cultured compound addition group as compared to the 2D-cultured control group. Therefrom it was clarified that the secretion of the paracrine factor can be promoted most by adding the compound k-1:B-1 to the 3D-cultured mesenchymal stem cells.

**[Table 5]**

| (relative value of secretion amount (pg) per 10000 cells) | | |
|---|---|---|
| culture method | compound | STC-1 |
| 2D | DMSO | 1.00 |
| | k-1:B-1 | 2.67 |
| 3D | DMSO | 2.25 |
| | k-1:B-1 | 8.95 |

### [Example 3] paracrine factor release amount test under inflammation stimulation

### (recovery of culture supernatant)

Human bone marrow-derived mesenchymal stem cells (hMSC, manufactured by PromoCell) were precultured by a single layer culture method (2D) using Mesenchymal Stem Cell Growth Medium 2 (manufactured by PromoCell). hMSCs were suspended in the same medium. In three-dimensional culture method (3D), hMSCs were seeded at 5×10⁵ cells/2 mL/well on a cell-non-adhesive 6-well EZSPHERE plate (manufactured by AGC TECHNO GLASS, #4810-900) having a depression on the bottom surface. In 2D, hMSCs were seeded on a 6-well flat bottom plate (manufactured by Corning Incorporated, #3516) at 5×10⁵ cells/2 mL/well. Continuously, compound k-1:J-1 dissolved in DMSO was added at 2 µL/well to a final concentration of 5 µM, and the mixture was cultured in an incubator at 37°C, 5% CO₂ for 48 hr. As a control, DMSO was added at a final concentration of 0.05%. After 48 hr, the medium was exchanged with medium supplemented only with 10 ng/mL TNFα (R&D Systems, #210-TA-020), and the cells were cultured in an incubator at 37°C, 5% CO₂. After 48 hr, the culture supernatant was recovered. The number of cells at the time of recovery of the culture supernatant was measured by dispersing the cells in single cells using Detach Kit (manufactured by PromoCell), suspending a part thereof in Trypan Blue (manufactured by Wako Pure Chemical Industries, Ltd.) and counting the number of viable cells using TC-20 (manufactured by BIO-RAD).

### (quantification using ELISA)

Tumor necrosis factor-inducible gene 6 protein (TSG-6), a cytokine which is one kind of paracrine factor contained in the culture supernatant, was quantified using a plate reader. For the quantification, TSG-6 antibody (Santa Cruz, #sc-65886) was immobilized on a NuncMaxisorp (Thermo Fisher Scientific, #44-2404-21) plate, reacted with a culture supernatant sample, and detected using biotinylated TSG-6 antibody (R&D Systems, #BAF-2104), streptavidin-HRP (Abcam, #ab7403), a substrate solution (KPL, #52-00-03), and a reaction quenching liquid (KPL, #50-85-06). The calibration curve was prepared using recombinant human TSG-6 protein (R&D Systems, #2104-TS).

### (quantification using flow cytometer)

Monocyte Chemotactic Protein-1 (MCP-1), a cytokine which is one kind of paracrine factor contained in the culture supernatant, was quantified using a flow cytometer. The quantification analysis was performed using BD™ CBA Flex Set (manufactured by BD 558287) according to the protocol of BD.

The concentration of TSG-6 and MCP-1 in the culture supernatant was normalized by the cell count, and the relative values for the control group cultured in 2D are shown in Table 6. As a result, the amount of secreted TSG-6 increased in 3D culture as compared to 2D culture. In addition, the amount of secreted TSG-6 increased in both 2D culture and 3D culture by adding compound k-1:J-1, and the amount of secreted TSG-6 increased 3.21-fold in the 3D-cultured compound addition group as compared to the 2D-cultured control group. The amount of secreted MCP-1 increased only in the 3D-cultured compound addition group. While it is a known fact that TNFα stimulation promotes the production of TSG-6 and MCP-1, it was clarified that the responsiveness to TNFα stimulation is improved and the secretion of the paracrine factor can be promoted by adding the compound k-1:J-1 to the 3D-cultured mesenchymal stem cells. This indicates that the compound k-1:J-1 enhances responsiveness to inflammation stimulation and releases a large amount of anti-inflammatory cytokines, and that compound k-1:J-1 is extremely useful as an anti-inflammatory therapeutic agent.

**[Table 6]**

| (relative value of secretion amount (pg) per 10000 cells) | | | |
|---|---|---|---|
| culture method | compound | TSG-1 | MCP-1 |
| 2D | DMSO | 1.00 | 1.00 |
| | k-1:J-1 | 1.56 | 0.85 |
| 3D | DMSO | 2.53 | 1.07 |
| | k-1:J-1 | 3.21 | 1.41 |

### [Example 4] paracrine factor release amount test 3

### (recovery of culture supernatant)

Human bone marrow-derived mesenchymal stem cells (hMSC, manufactured by PromoCell) were precultured by a single layer culture method (2D) using Mesenchymal Stem Cell Growth Medium 2 (manufactured by PromoCell). hMSCs were suspended in the same medium. In three-dimensional culture method (3D), hMSCs were seeded at 5×10⁵ cells/2 mL/well on a cell-non-adhesive 6-well EZSPHERE plate (manufactured by AGC TECHNO GLASS, #4810-900) having a depression on the bottom surface. In 2D, hMSCs were seeded on a 6-well flat bottom plate (manufactured by Corning Incorporated, #3516) at 5×10⁵ cells/2 mL/well. Continuously, compounds k-1:B-1, k-1:D-1, k-1:J-1 dissolved in DMSO were added at 2 µL/well to a final concentration of 5 µM, and the mixture was cultured in an incubator at 37°C, 5% CO₂ for 48 hr. As a control, DMSO was added at a final concentration of 0.05%. After 48 hr, the medium was exchanged with a medium not containing the compound, and the cells were cultured in an incubator at 37°C, 5% CO₂. After 48 hr, the culture supernatant was recovered. The number of cells at the time of recovery of the culture supernatant was measured by dispersing the cells in single cells using Detach Kit (manufactured by PromoCell), suspending a part thereof in Trypan Blue (manufactured by Wako Pure Chemical Industries, Ltd.) and counting the number of viable cells using TC-20 (manufactured by BIO-RAD).

### (quantification using ELISA)

Vascular endothelial growth factor (VEGF), a cytokine which is one kind of paracrine factor contained in the culture supernatant, was quantified using a plate reader. For the quantification, Human VEGF ELISA Kit (manufactured by Abcam, #ab100662) was used.

The concentration of VEGF in the culture supernatant was normalized by the cell count, and the relative values for the control group cultured in 2D are shown in Table 7. As a result, the secretion amount increased in both 2D culture and 3D culture by adding compounds k-1:B-1, k-1:D-1, and compound k-1:J-1. Therefrom it was clarified that the secretion of the paracrine factor can be promoted by adding the compounds k-1:B-1, k-1:D-1, and compound k-1:J-1 to the mesenchymal stem cells.

**[Table 7]**

| (relative value of secretion amount (pg) per 10000 cells) | | |
|---|---|---|
| culture method | compound | VEGF |
| 2D | DMSO | 1.00 |
| | k-1:B-1 | 4.40 |
| 3D | DMSO | 1.74 |
| | k-1:B-1 | 3.59 |
| | k-1:D-1 | 3.27 |
| | k-1:J-1 | 6.18 |

### [Example 5] paracrine factor release amount test under 3D culture conditions

### (recovery of culture supernatant)

Human bone marrow-derived mesenchymal stem cells (hMSC, manufactured by PromoCell) were precultured by a single layer culture method (2D) using Mesenchymal Stem Cell GrowthMedium 2 (manufactured by PromoCell). hMSCs were suspended in the same medium. hMSCs were seeded at 20000 cells/250 µL/well on a cell-non-adhesive 24-well EZSPHERE plate (manufactured by AGC TECHNO GLASS, #4820-900SP) having a depression on the bottom surface. Continuously, the specific compound used in the present invention dissolved in DMSO was added at 250 µL/well to a final concentration of 10 µM, and the mixture was cultured in an incubator at 37°C, 5% CO₂ for 72 hr. As a control, DMSO was added to a final concentration of 0.1%. After 72 hr, the medium was exchanged with a medium not containing the specific compound, and the cells were cultured in an incubator at 37°C, 5% CO₂. After 24 hr, the culture supernatant was recovered. The number of cells at the time of recovery of the culture supernatant was normalized using the total protein amount measured by protein assay BCA kit (manufactured by FUJIFILM Wako Pure Chemical Corporation, #297-73101).

### (quantification using ELISA)

Vascular endothelial growth factor (VEGF) and Angiogenin (ANG), cytokines which are each one kind of paracrine factor contained in the culture supernatant, were quantified using a plate reader. For the quantification, Human VEGF ELISA Kit (manufactured by Abcam, #ab100662), and Human Angiogenin ELISA Kit (manufactured by Abcam, #ab99970) were respectively used.

The concentrations of VEGF and ANG in the culture supernatant were normalized by the total protein amount, and the relative values for the control group were calculated. Compound numbers with increased relative amount of secreted cytokines are described below. As a result of this test, it was clarified that the specific compounds used in the present invention promotes the secretion of paracrine factor.

Compound numbers with relative amount of secreted VEGF increased by 1.2 times or more:
k-1:B-1, k-1:J-1, A-004, GA-002A, A-004A

Compound numbers with relative amount of secreted VEGF increased by 2 times or more:
k-1:B-1, A-004A

Compound numbers with relative amount of secreted ANG increased by 2 times or more:
k-1:H-1, k-1:B-1, k-1:D-1, k-1:J-1, A-004, GA-002A, A-004A, A-007, GA-005A

Compound numbers with relative amount of secreted ANG increased by 4 times or more:
k-1:H-1, k-1:B-1, k-1:D-1, A-004A

### [Industrial Applicability]

According to the present invention, the production of paracrine factor in cells can be promoted very easily and efficiently. In addition, the cells produced using the present invention show promoted production and secretion of paracrine factor and are considered to be very suitable as cells for transplantation. In addition, the cells (e.g., mesenchymal stem cells) produced using the present invention show remarkably promoted production and secretion of a plurality of anti-inflammatory proteins and angiogenesis-promoting proteins among the paracrine factors. Therefore, the cells (e.g., mesenchymal stem cells) produced using the present invention are preferably used for the treatment of anti-inflammatory diseases and ischemic diseases. From the above, the present invention is extremely beneficial in the cell culture field and the medical field.

This application is based on a patent application No. 2018-185799 filed in Japan (filing date: September 28, 2018), the contents of which are incorporated in full herein.

## Claims

1. A medium additive for promoting production of a paracrine factor, comprising a compound represented by the following formula (I): 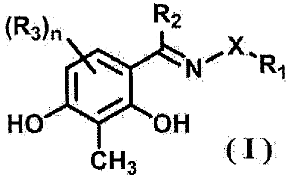 wherein, X is -NHCO-, R₁ is -Y-NH-Z-Ar wherein Y and Z are each a single bond or an alkylene group having 1 - 6 carbon atoms and optionally having substituent(s), and Ar is an aryl group or a heteroaryl group optionally having substituent(s), R₂ is an alkyl group having 1 - 6 carbon atoms and optionally having substituent(s), R₃ is a hydroxyl group, and n is an integer of 0, 1 or 2,
or a salt thereof.

2. The additive according to claim 1, wherein R₂ is a methyl group, an ethyl group, or an isobutyl group,
n is 0,
Ar is a phenyl group optionally substituted by a hydroxyl group or a methyl group, and
Y is a methylene group optionally substituted by a methyl group or an ethyl group.

3. The additive according to claim 1 or 2, wherein the compound is a compound selected from the group consisting of the following: 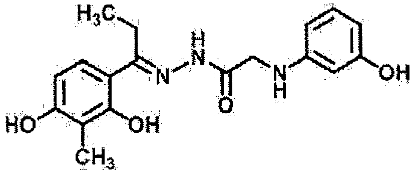 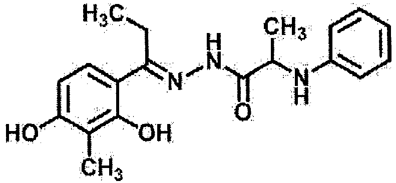 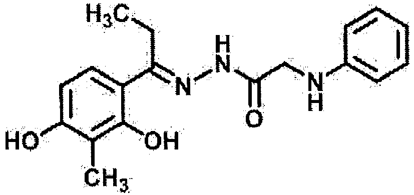 and 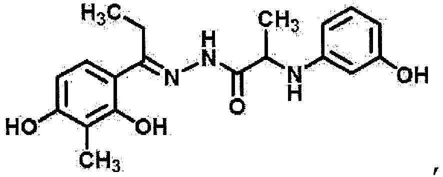 or a salt thereof.

4. The additive according to claim 1, wherein the compound is a compound represented by: 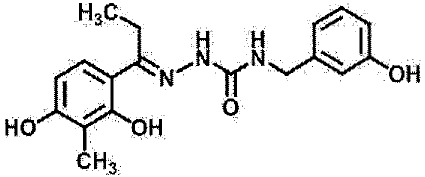 or a salt thereof.

5. The additive according to claim 1, wherein the compound is a compound represented by: 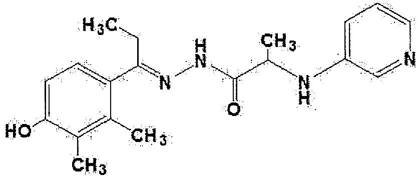 or 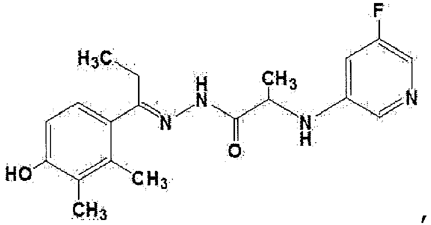 or a salt thereof.

6. The additive according to any one of claims 1 to 5, wherein the paracrine factor is at least one type of protein selected from the group consisting of an anti-inflammatory protein and an angiogenesis promoting protein.

7. The additive according to claim 6, wherein the anti-inflammatory protein is at least one type of protein selected from the group consisting of TNF-stimulated gene 6 protein (TSG-6) and Stanniocalcin-1 (STC-1).

8. The additive according to claim 6, wherein the angiogenesis promoting protein is at least one type of protein selected from the group consisting of Angiogenin (ANG), Epidermal Growth Factor (EGF), Monocyte Chemotactic Protein-1 (MCP-1), Epithelial-derived neutrophil-activating peptide 78 (ENA-78), Basic fibroblast growth factor (bFGF), Interleukin-6 (IL-6), Interleukin-8 (IL-8), Vascular endothelial growth factor (VEGF), Vascular endothelial growth factor-D (VEGF-D), Tissue inhibitors of matrix metalloproteinase (TIMP), Platelet-Derived Growth Factor (PDGF), and Transforming growth factor-β (TGF-β).

9. The additive according to any one of claims 1 to 8, wherein the cell is a mesenchymal stem cell.

10. A cell culture medium comprising the additive according to any one of claims 1 to 9.

11. A method for producing a cell showing promoted production of a paracrine factor, comprising culturing cells in a medium comprising a compound represented by the following formula (I): 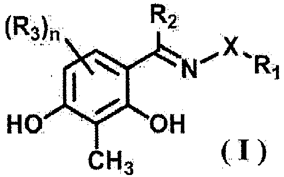 wherein, X is -NHCO-, R₁ is -Y-NH-Z-Ar wherein Y and Z are each a single bond or an alkylene group having 1 - 6 carbon atoms and optionally having substituent(s), and Ar is an aryl group or a heteroaryl group optionally having substituent(s), R₂ is an alkyl group having 1 - 6 carbon atoms and optionally having substituent(s), R₃ is a hydroxyl group, and n is an integer of 0, 1 or 2,
or a salt thereof.

12. The method according to claim 11, wherein R₂ is a methyl group, an ethyl group, or an isobutyl group,
n is 0,
Ar is a phenyl group optionally substituted by a hydroxyl group or a methyl group, and
Y is a methylene group optionally substituted by a methyl group or an ethyl group.

13. The method according to claim 11 or 12, wherein the compound represented by the formula (I) is a compound selected from the group consisting of the following or a salt thereof: 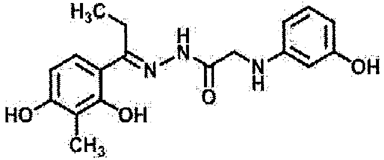 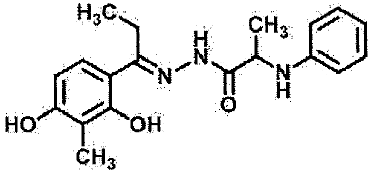 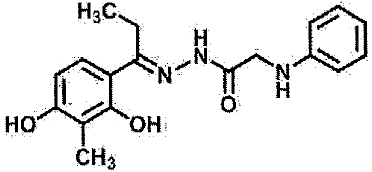 and 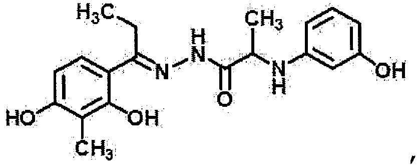 or a salt thereof.

14. The method according to claim 11, wherein the compound is a compound represented by: 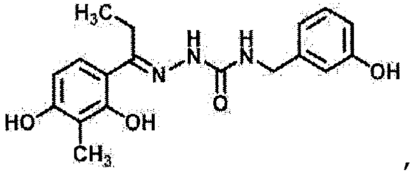 or a salt thereof.

15. The method according to claim 11, wherein the compound is a compound represented by: 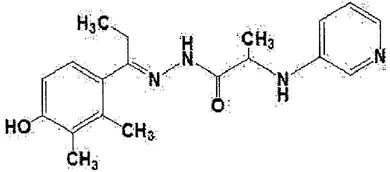 or 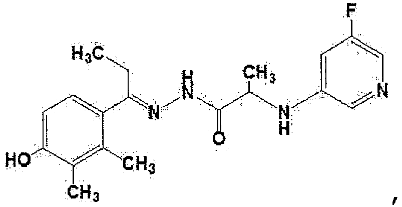 or a salt thereof.

16. The method according to any one of claims 11 to 15, wherein the paracrine factor is at least one type of protein selected from the group consisting of an anti-inflammatory protein and an angiogenesis promoting protein.

17. The method according to claim 16, wherein the anti-inflammatory protein is at least one type of protein selected from the group consisting of TNF-stimulated gene 6 protein (TSG-6) and Stanniocalcin-1 (STC-1).

18. The method according to claim 16, wherein the angiogenesis promoting protein is at least one type of protein selected from the group consisting of Angiogenin (ANG), Epidermal Growth Factor (EGF), Monocyte Chemotactic Protein-1 (MCP-1), Epithelial-derived neutrophil-activating peptide 78 (ENA-78), Basic fibroblast growth factor (bFGF), Interleukin-6 (IL-6), Interleukin-8 (IL-8), Vascular endothelial growth factor (VEGF), Vascular endothelial growth factor-D (VEGF-D), Tissue inhibitors of matrix metalloproteinase (TIMP), Platelet-Derived Growth Factor (PDGF), and Transforming growth factor-β (TGF-β).

19. The method according to any one of claims 11 to 18, wherein the cell is a mesenchymal stem cell.

20. The method according to any one of claims 11 to 19, wherein the culture is performed in a three-dimensional culture.

21. A composition for in vivo transplantation, comprising a cell produced by the method according to any one of claims 11 to 20.

22. A composition for treating an inflammatory disease or ischemic disease, comprising a cell produced by the method according to claim 19 or 20.

23. The composition according to claim 22, wherein the inflammatory disease is selected from the group consisting of inflammatory bowel disease, ulcerative colitis, Crohn's disease, nephritis, acute nephritis, chronic nephritis, glomerulonephritis, IgA nephropathy, diabetic nephropathy, membranous nephropathy, hydronephrosis, contrast nephropathy, pyelonephritis, renal failure, interstitial nephritis, renal disorder, nephrotic syndrome, hypertensive nephrosclerosis, diabetic glomerulosclerosis, renal calculus, amyloid kidney, renal vein thrombosis, Alport syndrome, hepatitis, cirrhosis, pancreatitis, pneumonia, sinusitis, rhinitis, arthritis, knee osteoarthritis, hand osteoarthritis, hip osteoarthritis, ankle osteoarthritis, hip osteoarthritis, rheumatoid arthritis, periodic fever, aphthous stomatitis, pharyngitis, mucocutaneous lymphnode syndrome, adult onset Still's disease, Behcet's disease, gout, pseudogout, Schnitzler syndrome, chronic relapsing multifocal osteomyelitis, Cryopyrin-associated periodic syndrome, familial cold urticaria, Muckle-Wells syndrome, Chronic infantile neurologic cutaneous, and articular syndrome, Neonatal onset multisystem inflammatory disease, tumor necrosis factor (TNF) receptor associated periodic syndrome, Hyper-IgD syndrome, Blau syndrome, Early-onset sarcoidosis, familial Mediterranean fever, pyogenic arthritis, pyoderma gangrenosum, contusion, Nakajo-Nishimura syndrome, Majeed syndrome, NLRP12-associated periodic syndrome, deficiency of interleukin-1 receptor antagonist deficiency of interleukin-1 receptor antagonist, deficiency of interleukin-36 receptor antagonist, auto-inflammation and phospholipase Cγ2-associated antibody deficiency and immune dysregulation syndrome, HOIL-1 deficiency, SLC29A3 deficiency, CARD14 abnormality, adenosine deaminase 2 deficiency, STING-Associated Vasculopathy with Onset in Infancy, and NLRC4 abnormality.

24. The composition according to claim 20, wherein the ischemic disease is selected from the group consisting of angina pectoris, myocardial infarction, takotsubo cardiomyopathy, central pulmonary edema, cerebral infarction, ischemic cerebral apoplexy, arteriosclerosis obliterans, and severe lower leg ischemia.

25. A method for producing a paracrine factor, comprising the following steps:
(1) a step of culturing a cell in the medium according to claim 10, and
(2) a step of recovering a supernatant from the medium used in (1).

26. The method according to claim 25, wherein the cell is a mesenchymal stem cell.

27. The method according to claim 25 or 26, wherein the paracrine factor is at least one type of protein selected from the group consisting of an anti-inflammatory protein and an angiogenesis promoting protein.
